(19) Europäisches Patentamt
European Patent Office
Office européen des brevets

(11) EP 2 328 949 B1

(12) **EUROPEAN PATENT SPECIFICATION**

(45) Date of publication and mention of the grant of the patent:
**23.04.2014 Bulletin 2014/17**

(21) Application number: **09792019.3**

(22) Date of filing: **28.08.2009**

(51) Int Cl.:
***C08F 283/12*** (2006.01) ***A61K 8/81*** (2006.01)
***A61K 8/89*** (2006.01) ***A61K 8/891*** (2006.01)
***A61K 8/898*** (2006.01) ***A61K 8/91*** (2006.01)
***A61Q 19/00*** (2006.01) ***C08L 51/08*** (2006.01)
***C08L 33/08*** (2006.01) ***C08L 33/12*** (2006.01)
***C08L 83/04*** (2006.01) ***C08L 83/08*** (2006.01)

(86) International application number:
**PCT/US2009/055268**

(87) International publication number:
**WO 2010/025311 (04.03.2010 Gazette 2010/09)**

(54) **SILICONE-ORGANIC HYBRID EMULSIONS IN PERSONAL CARE APPLICATIONS**

SILIKON-ORGANISCHE HYBRIDEMULSIONEN IN KÖRPERPFLEGEANWENDUNGEN

ÉMULSIONS HYBRIDES SILICONE-ORGANIQUE DANS DES APPLICATIONS DE SOIN PERSONNEL

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.08.2008 US 92798 P**

(43) Date of publication of application:
**08.06.2011 Bulletin 2011/23**

(73) Proprietor: **Dow Corning Corporation**
**Midland, Michigan 48686-0994 (US)**

(72) Inventors:
- **CANFIELD, Lylenette**
  **Midland, MI 48640 (US)**
- **JOHNSON, Bethany**
  **Midland, MI 48642 (US)**
- **MEYERS, Deborah**
  **Midland, MI 48640 (US)**
- **SCAVUZZO, Concettina**
  **B-7160 Chap.-Lez-herlaimont (BE)**
- **SHOPE, Marilyn, Patrick**
  **Sanford, MI 48657 (US)**
- **SWANTON, Brian, Jeffery**
  **Sagianw, MI 48603 (US)**
- **VINCENT, Anne, Marie**
  **B-6210 Les Bons Villers (BE)**

(74) Representative: **Thomson, James B. et al**
**Murgitroyd & Company**
**Scotland House**
**165-169 Scotland Street**
**Glasgow G5 8PL (GB)**

(56) References cited:
**EP-A1- 1 062 936**
**WO-A1-2005/056682**
**WO-A2-2004/100862**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 328 949 B1

## Description

### Technical Field

This disclosure relates to the use of silicone polymer and organic polymer containing alloy and/or hybrid emulsions in personal care products. The silicone organic hybrid emulsions are particularly useful in hair care formulations to simultaneously provide conditioning and styling benefits. The silicone organic hybrid emulsions are also useful in color cosmetic formulations to provide shine and wash-off resistance benefits.

### Background

Emulsions of silicone organic alloys or hybrid emulsions of silicone and organic polymers have been disclosed in WO 2005/056682. Such emulsions provide an improvement upon solvent-based polymers by using emulsion polymerization of both the silicone polymer phase and the organic polymer phase, thus eliminating the need to dissolve the polymer in a solvent, and allowing the solid polymer to be added to personal care formulations as a low viscosity liquid. Such emulsions also are more cost effective than silicone-graft-organic polymers or silicone-block-organic polymers.

In today's hair care market, there continues to exist unmet needs for flexible styling benefits in hair care compositions. For example, when using organic resins to style hair, the hair tends to become rigid or brittle. In addition, organic resins do not typically provide conditioning properties to hair.

The present inventors have discovered that emulsions containing a silicone organic alloy or hybrid emulsion are useful for imparting conditioning and styling benefits to hair. That is, the emulsions of the present disclosure demonstrate flexible styling benefits as well as the provision of conditioning properties to hair. As used herein "silicone alloy" means an immiscible mixture of two polymers, i.e., a silicone polymer and an organic polymer within each polymer particle. In addition, the present emulsions combine the unique characteristics of silicone polymers, with the hydrophilic and/or hydrophobic film forming, barrier and other properties of organic polymers, and in that sense can be considered hybrid emulsions. The silicone organic hybrid emulsions are also particularly useful in color cosmetic formulations to provide shine and wash-off resistance benefits.

### Summary

The present disclosure relates to personal care compositions containing silicone organic alloys or hybrid emulsions of silicone and organic polymers. Silicone organic hybrid emulsions are disclosed in WO 2005/056682. The silicone organic hybrid emulsions of the invention are selected from an aminofunctional silicone - organic hybrid emulsion, as disclosed herein. Representative personal care compositions include; a color cosmetic, a lipstick, a foundation, a shampoo, a hair conditioner, a hair fixative, a hair styling aid, a hair colorant, a hair relaxer, a shower gel, a skin moisturizer, a skin conditioner, a body conditioner, a sun protection product, an antiperspirant, and a deodorant. The silicone organic hybrid emulsions are particularly useful for imparting conditioning and styling benefits to hair. The silicone organic hybrid emulsions are also useful in color cosmetic formulations to provide shine and wash-off resistance benefits.

### Detailed Description

This disclosure is directed to personal care compositions containing a polymer alloy and/or hybrid emulsion compositions containing a silicone polymer and an organic polymer. As used herein, (i) silicone polymer means an organopolysiloxane containing siloxane units independently selected from ($R_3SiO_{1/2}$), ($R_2SiO_{2/2}$), ($RSiO_{1.5}$), or ($SiO_2$) siloxy units (commonly known as M, D, T, and Q siloxy units respectively, where R typically represents a saturated alkyl group containing 1-6 carbon atoms such as methyl, or an aryl group containing 6-10 carbon atoms such as phenyl, but R can optionally contain unsaturated groups such as vinyl or allyl, or functional groups such as amino or mercapto); (ii) organic polymer means a polymer free of silicon atoms; and (iii) polymer alloy and/or hybrid emulsion means an aqueous oil-in-water (O/W) emulsion containing an immiscible mixture of linear silicone polymers and organic polymers.

The silicone organic hybrid emulsions of the invention are selected from an aminofunctional silicone - organic hybrid emulsion, as disclosed herein.

Polymer alloy emulsions of the invention can be prepared by combining silicone polymer emulsions prepared by emulsion polymerization, and organic polymer emulsions prepared by free radical emulsion polymerization of acrylate monomers and/or other organic monomers. The silicone polymer emulsions are emulsions containing silicone polymer particles having an average diameter 30-500 nanometers, and in which the viscosity of the internal phase of the silicone emulsion is 2,000-10,000,000 centistoke ($mm^2/s$) at 25°C. The polymer alloy emulsion compositions are substantially two-phase emulsion particles composed of a silicone phase and an organic phase, in which there exists no grafting monomers in the composition, and in which there is no chemical crosslinking between the silicone and organic polymer

chains in the emulsion. Rather, the existing chains were entangled at the silicone-organic interface within the particles. The silicone polymer and organic polymer emulsion compositions typically contained a 50:50 weight ratio of the silicone polymer emulsion solids to the acrylate polymer emulsion solids, but any weight ratio of solids can be prepared, depending on the desired properties of the final emulsion.

One process embodiment for preparing polymer alloy emulsions is a *semi-continuous process* while another process embodiment for preparing polymer alloy emulsions is a *monomer swell process.* According to the semi-continuous process, the procedure commences with the preparation of a silicone emulsion by emulsion polymerization as a first stage. In the second stage, the organic monomer(s) are then introduced to a reactor continuously over a period of several hours at a temperature of 80-90 °C. A water-soluble free radical initiator such as sodium persulfate is then added to the reactor in a separate stream over a period of several hours during addition of the organic monomer(s). In the monomer swell process, it also commences with preparation of a silicone emulsion by emulsion polymerization. However, the organic monomer(s) and an organic free radical azo-type initiator are introduced to the reactor simultaneously and allowed to swell the silicone emulsion particles for a brief period of time. Heating is then increased to activate the initiator, causing the organic monomer(s) to polymerize within the silicone emulsion particles.

Thermal or redox initiation processes are used in the preparation of the organic polymer phase. Conventional thermal free radical initiators which can be used include hydrogen peroxide, sodium peroxide, potassium peroxide, t-butyl hydroperoxide, cumene hydroperoxide, ammonium and/or alkali metal persulfates, sodium perborate, perphosphoric acid and salts thereof, potassium permanganate, and ammonium or alkali metal salts of peroxydisulfuric acid. These initiators are typically used at a level of 0.01-3.0 percent by weight based on the total weight of monomer. Redox initiators which can be used are typically an oxidant plus a reducing agent in combinations effective to generate free radicals, including the same free radical initiators listed above as the oxidant; and a suitable reductant such as sodium sulfoxylate formaldehyde, ascorbic acid, isoascorbic acid, alkali metal and ammonium salts of sulfur-containing acids such as sodium sulfite, bisulfite, thiosulfate, hydrosulfite, sulfide, hydrosulfide or dithionite, formamidine sulfinic acid, hydroxymethane sulfonic acid, acetone bisulfite; amines such as ethanolamine, glycolic acid, glyoxylic acid hydrate, lactic acid, glyceric acid, malic acid, tartaric acid, as well as salts of the preceding acids which may be used.

Redox reaction catalyzing metal salts of iron, copper, manganese, silver, platinum, vanadium, nickel, chromium, palladium, or cobalt, may optionally be used. The initiator or initiator system can be added in one or more additions, continuously, linearly, or not, over the reaction period, or as combinations thereof. Several azo-type organic free radical initiators which can be used in the monomer swell process such as azobis-isobutyronitrile and azobispropionitrile, which are sold under the trademark VAZO® by E.I. du Pont de Nemours Company.

As used herein, the term *emulsion polymerization* refers to its accepted meaning in the art, for example, any of the polymerization processes represented by processes described in US Patents US 2,891,920 (June 23, 1959), US 3,294,725 (December 27, 1966), US 4,999,398 (March 12, 1991), US 5,502,105 (March 26, 1996), US 5,661,215 (August 26, 1997), and US 6,316,541 (November 13,2001).

Emulsion polymerization processes useful in the present invention are typically carried out at a temperature in the range of 25-100 °C, preferably 50-95 °C, and in the case of silicone emulsion polymerization, most typically involve opening of the ring of a volatile siloxane oligomer using an acid or a base catalyst in the presence of water. Upon opening of the ring, siloxanes with terminal hydroxy groups are formed. These siloxanes then react with one another by a condensation reaction to form the siloxane polymer.

A simplified representation of the process chemistry is shown below for a volatile siloxane oligomer such as octamethylcyclotetrasiloxane, in which Me represents $CH_3$;

$$(Me_2SiO)_4 + H_2O + Catalyst \rightarrow HOMe_2SiOMe_2SiOMe_2SiOSiMe_2OH \rightarrow HOMe_2SiOMe_2SiOMe_2SiOSiMe_2OH + HOMe_2SiOMe_2SiOMe_2SiOSiMe_2OH \rightarrow HOMe_2SiO(Me_2SiO)_6SiMe_2OH + H_2O.$$

It is also feasible to produce linear siloxane polymers through the emulsion polymerization reaction by using oligomers in the reaction medium that contain silanol groups. Such hydroxy endblocked siloxane oligomers generally comprise a silanol terminated polydimethylsiloxane with a degree of polymerization of 1-7. A process similar to the above process occurs in instances when the oligomers consist of siloxanes containing silanol groups: $HOMe_2SiO(Me_2SiO)_{6-15}SiMe_2OH + H_2O + Catalyst + Shear \rightarrow HOMe_2SiO(Me_2SiO)_{600-1500}SiMe_2OH + H_2O$. Siloxane polymers of higher molecular weight can be obtained by allowing both of the processes to continue.

Aminofunctional silicones may be produced by the addition of an amino functional silane during the emulsion polymerizations involving either the ring opening of a cyclic siloxane or the condensation of a hydroxy endblocked siloxane oligomer, as discussed above. The amino functional silane may be an alkoxy silane of the general formula $R^N{}_aSi(OR)_{(4-a)}$ In the formula, $R^N$ represents an amino-functional organic group. The value of subscript "a" is 1 or 2, and R' represents an alkyl group having 1-6 carbon atoms. The amino-functional organic group is designated in the formulas herein as $R^N$ and is illustrated by groups having the formula; $-R^1NHR^2$, $-R^1NR^2{}_2$, or $-R^1NHR^1NHR^2$, wherein each $R^1$ is independently a divalent hydrocarbon group having at least 2 carbon atoms, and $R^2$ is hydrogen or an alkyl group

containing 1 to 4 carbon atoms.

Some examples of suitable amino-functional hydrocarbon groups are;-$CH_2CH_2NH_2$,

- $CH_2CH_2CH_2NH_2$, -$CH_2CHCH_3NH$, $CH_2CH_2CH_2CH_2NH_2$,
- $CH_2CH_2CH_2CH_2CH_2NH_2$, -$CH_2CH_2CH_2CH_2CH_2CH_2NH_2$,
- $CH_2CH_2NHCH_3$, -$CH_2CH_2CH_2NHCH_3$, -$CH_2(CH_3)CHCH_2NHCH_3$,
- $CH_2CH_2CH_2CH_2NHCH_3$, - $CH_2CH_2CH_2NHCH_2CH_2NH_2$,
- $CH_2CH_2CH_2NHCH_2CH_2CH_2NH_2$, -$CH_2CH_2CH_2CH_2NHCH_2CH_2CH_2CH_2NH_2$,
- $CH_2CH_2NHCH_2CH_2NHCH_3$, -$CH_2CH_2CH_2NHCH_2CH_2CH_2NHCH_3$,
- $CH_2CH_2CH_2CH_2NHCH_2CH_2CH_2CH_2NHCH_3$, and
- $CH_2CH_2NHCH_2CH_2NHCH_2CH_2CH_2CH_3$. Typically, the amino functional group is
- $CH_2CH_2CH_2NHCH_2CH_2NH_2$.

Aminofunctional silanes are known, and many are available commercially such as Dow Corning® Z-6020 (Dow Coming Corporation, Midland MI 48642).

In addition, linear siloxane polymers can be prepared through the emulsion polymerization reaction by using oligomers in the reaction medium that contain vinyl and hydrogen groups that react by chain extension. Such chain extension reactions with polysiloxanes involve the hydrosilylation reaction in which Si-H groups reacts with aliphatically unsaturated groups in the presence of platinum or rhodium containing catalysts. Alternatively, such reactions can involve reactions between Si-OH groups with alkoxy groups in alkoxysilanes, silicates, or alkoxysiloxanes, in the presence of a metal containing catalyst. Still other reactions can involve reaction of an Si-OH group with $CH_3COOSi$- groups in the presence of water; or the reaction of Si-OH groups with an Si-H group in the presence of a metal containing catalyst.

A typical reaction consists of a dimethylvinylsiloxy terminated polydimethylsiloxane (Vi-PDMS) having a viscosity of 7,000-12,000 $mm^2$ /sec at 25 °C.; 0.9 parts of a liquid organohydrogen polysiloxane having an average formula of $Me_2HSiO(Me_2SiO)_{20}SiMe_2H$ and containing 0.16-0.20 percent Si-H; and 0.015 parts of platinum in a platinum catalyst material. The following process illustrates what occurs in instances where the oligomers consist of siloxanes containing vinyl and hydrogen groups:

Vi-PDMS + $Me_2HSiO(Me_2SiO)_{20}SiMe_2H$ + surfactant + $H_2O$ + Metal Catalyst + Shear $\rightarrow$ Vi-$Me_2SiO(Me_2SiO)_{10,000\text{-}270,000}SiHMe_2$ + $H_2O$. Siloxane polymers of very high molecular weight can be obtained by this process.

Catalysts used in such processes include strong mineral acids such as hydrochloric acid; strong alkaline catalysts such as sodium hydroxide; quaternary ammonium hydroxides; surface active sulfonic acids such as dodecylbenzene sulfonic acid and the sodium salts thereof; silanolates; and organosilanolates. Other examples of suitable catalysts can be found in US Patents 2,891,920; 3,294,725; 4,999,398; 5,502,105; 5,661,215; and 6,316,541.

Some representative cyclic siloxanes are hexamethylcyclotrisiloxane, a solid at room temperature, with a boiling point of 134 °C and formula $(Me_2SiO)_3$; octamethylcyclotetrasiloxane ($D_4$) with a boiling point of 176 °C, viscosity of 2.3 $mm^2$/s, and formula $(Me_2SiO)_4$; decamethylcyclopentasiloxane with a boiling point of 210 °C, viscosity of 3.87 $mm^2$/s, and formula $(Me_2SiO)_5$; and dodecamethylcyclohexasiloxane with a boiling point of 245 °C, viscosity of 6.62 $mm^2$/s, and formula $(Me_2SiO)_6$. It is possible to use other types of cyclic siloxanes such as cyclic siloxanes containing saturated alkyl groups with 2-30 carbon atoms, or cyclic siloxanes in which Si-H groups are used in place of one or more of the Si-Me groups present.

The emulsions containing the silicone polymer useful in the present invention can contain anionic surfactants, cationic surfactants, and nonionic surfactants. The anionic surfactants include sulfonic acids and their salt derivatives. Some examples of anionic surfactants are alkali metal sulfosuccinates; sulfonated glyceryl esters of fatty acids such as sulfonated monoglycerides of coconut oil acids; salts of sulfonated monovalent alcohol esters such as sodium oleyl isothionate; amides of amino sulfonic acids such as the sodium salt of oleyl methyl tauride; sulfonated products of fatty acid nitriles such as palmitonitrile sulfonate; sulfonated aromatic hydrocarbons such as sodium alpha-naphthalene monosulfonate; condensation products of naphthalene sulfonic acids with formaldehyde; sodium octahydro anthracene sulfonate; alkali metal alkyl sulfates; ether sulfates having alkyl groups of eight or more carbon atoms such as sodium lauryl ether sulfate; and alkylaryl sulfonates having one or more alkyl groups of eight or more carbon atoms such as neutral salts of hexadecylbenzene sulfonic acid and $C_{20}$ alkylbenzene sulfonic acid.

Commercial anionic surfactants which can be used include the sodium salt of dodecylbenzene sulfonic acid sold under the trademark SIPONATE® DS-10 by Alcolac Inc., Baltimore, Maryland; sodium n-hexadecyl diphenyloxide disulfonate sold under the trademark DOWFAX® 8390 by The Dow Chemical Company, Midland, Michigan; the sodium salt of a secondary alkane sulfonate sold under the trademark HOSTAPUR® SAS 60 by Clariant Corporation, Charlotte, North Carolina; N-acyl taurates such as sodium N-lauroyl methyl taurate sold under the trademark NIKKOL LMT® by Nikko Chemicals Company, Ltd., Tokyo, Japan; and linear alkyl benzene sulfonic acids sold under the trademark BIO-SOFT® S-100 by the Stepan Company, Northfield, Illinois. Compositions of the latter type such as dodecylbenzene

sulfonic acid, although a catalyst as noted above, can also function as the anionic surfactant when neutralized.

Cationic surfactants useful herein include compounds containing quaternary ammonium hydrophilic moieties in the molecule which are positively charged, such as quaternary ammonium salts represented by $R3R4R5R6N^+X^-$ where R3 to R6 are alkyl groups containing 1-30 carbon atoms, or alkyl groups derived from tallow, coconut oil, or soy; and X is halogen, i.e., chlorine or bromine. Dialkyl dimethyl ammonium salts can be used and are represented by $R7R8N^+(CH_3)_2X^-$ where R7 and R8 are alkyl groups containing 12-30 carbon atoms or alkyl groups derived from tallow, coconut oil, or soy; and X is halogen. Monoalkyl trimethyl ammonium salts can be used and are represented by $R9N^+(CH_3)_3X^-$ where R9 is an alkyl group containing 12-30 carbon atoms or an alkyl group derived from tallow, coconut oil, or soy; and X is halogen.

Representative quaternary ammonium salts are dodecyltrimethyl ammonium chloride/lauryltrimethyl ammonium chloride (LTAC), cetyltrimethyl ammonium chloride (CTAC), didodecyldimethyl ammonium bromide, dihexadecyldimethyl ammonium chloride, dihexadecyldimethyl ammonium bromide, dioctadecyldimethyl ammonium chloride, dieicosyldimethyl ammonium chloride, didocosyldimethyl ammonium chloride, dicoconutdimethyl ammonium chloride, ditallowdimethyl ammonium chloride, and ditallowdimethyl ammonium bromide. These quaternary ammonium salts are commercially available under trademarks such as ADOGEN®, ARQUAD®, TOMAH®, and VARIQUAT®.

Commercially available nonionic surfactants which can be used include compositions such as 2,6,8-trimethyl-4-nonyloxy polyethylene oxyethanols (6EO) and (10EO) sold under the trademarks TERGITOL® TMN-6 and TERGITOL® TMN-10; alkyleneoxy polyethylene oxyethanol ($C_{11-15}$ secondary alcohol ethoxylates 7EO, 9EO, and 15EO) sold under the trademarks TERGITOL® 15-S-7, TERGITOL® 15-S-9, TERGITOL® 15-S-15; other $C_{11-15}$ secondary alcohol ethoxylates sold under the trademarks TERGITOL® 15-S-12, 15-S-20, 15-S-30, 15-S-40; and octylphenoxy polyethoxy ethanol (40EO) sold under the trademark TRITON® X-405. All of these surfactants are sold by Union Carbide Corporation, Danbury, Connecticut.

Other useful commercial nonionic surfactants are nonylphenoxy polyethoxy ethanol (10EO) sold under the trademark MAKON® 10 by Stepan Company, Northfield, Illinois; polyoxyethylene 23 lauryl ether (Laureth-23) sold commercially under the trademark BRIJ® 35L by ICI Surfactants, Wilmington, Delaware; and RENEX® 30, a polyoxyethylene ether alcohol sold by ICI Surfactants, Wilmington, Delaware. When preparing silicone oil-in-water emulsions by emulsion polymerization, the presence of a nonionic surfactant is optional. However, when one is present, it is preferably present in combination with another surfactant such as an anionic or cationic surfactant.

Protective colloids, i.e., colloidal stabilizers, may be used, if desired, to enhance stability or to provide a specific rheological characteristic to the emulsion. As used herein, the terms *protective colloid* and/or *colloidal stabilizer* mean a nonionic molecule that is an effective agent for protecting charged colloidal particles in an aqueous media against flocculation. These compositions typically have a weight average molecular weight between 1,000-300,000 and are typically more hydrophilic than the composition of the first emulsion polymer, as measured by weight-averaged solubility parameters. Colloidal stabilizers which can be used include hydroxyethyl cellulose having a weight average molecular weight between 50,000-150,000; N-vinyl pyrrolidone; polyvinyl alcohol having a weight average molecular weight between 10,000-200,000; partially acetylated polyvinyl alcohol; carboxymethyl cellulose; gums such as gum arabic; starches; proteins; and mixtures thereof. Preferred colloidal stabilizers are hydroxethyl cellulose and polyvinyl alcohol.

Since emulsions are susceptible to microbiological contamination a preservative can be added. Representative preservatives, which can be used include formaldehyde; 1,3-dimethylol-5,5-dimethyl hydantoin, i.e., DMDM Hydantoin; 5-bromo-5-nitro-1,3-dioxane; methyl or propyl paraben; sorbic acid; imidazolidinyl urea; and KATHON® CG (5-chloro-2-methyl-4-isothiazolin-3-one).

Generally, the silicone emulsions contain a siloxane polymer concentration of 10 to 70 percent by weight based on the weight of the total emulsion, preferably 25 to 60 percent by weight. While emulsions containing less than 10 percent siloxane polymer content can be made, such emulsions hold little or no economic value. The surfactant is generally present at 0.05-30 percent by weight based on the weight of the total emulsion, preferably 0.1 to 20 percent by weight. Water and optional ingredients constitute the balance of the emulsion to 100 percent.

Various types of ethylenically unsaturated and/or vinyl containing organic monomers can be used for the organic phase including acrylates, methacrylates, substituted acrylates, substituted methacrylates, vinyl halides, fluorinated acrylates, and fluorinated methacrylates, for example. Some representative compositions include acrylate esters and methacrylate esters such as methyl acrylate, ethyl acrylate, butyl acrylate, 2-ethylhexyl acrylate, methyl methacrylate, decyl acrylate, lauryl acrylate, isodecyl methacrylate, lauryl methacrylate, and butyl methacrylate; substituted acrylates and methacrylates such as hydroxyethyl acrylate, perfluorooctyl acrylate, hydroxypropyl acrylate, hydroxypropyl methacrylate, and hydroxyethyl methacrylate; vinyl halides such as vinyl chloride, vinylidene chloride, and chloroprene; vinyl esters such as vinyl acetate and vinyl butyrate; vinyl pyrrolidone; conjugated dienes such as butadiene and isoprene; vinyl aromatic compounds such as styrene and divinyl benzene; vinyl monomers such as ethylene; acrylonitrile and methacrylonitrile; acrylamide, methacrylamide, and N-methylol acrylamide; and vinyl esters of monocarboxylic acids with up to 10 carbon atoms such as compositions sold under the trademarks VeoVa-9® and VeoVa-10® by Shell Chemical Oil Company, Houston, Texas.

In the polymerization technique for preparing the organic polymer phase, the monomer may be added in one or more additions which can be carried out continuously or linearly over the reaction period, or in combinations thereof. (i) The organic polymer phase may contain 0-5 percent by weight, preferably 0.5-2 percent by weight, based on the polymer weight, of a copolymerized monoethylenically unsaturated acid group containing monomer, based on the weight of the polymer, such as acrylic acid, methacrylic acid, crotonic acid, itaconic acid, fumaric acid, maleic acid, monomethyl itaconate, monomethyl fumarate, monobutyl fumarate, maleic anhydride, sulfoethyl methacrylate, and phosphoethyl methacrylate. (ii) The organic polymer phase may also contain 0-5 percent by weight, preferably 0-2 percent by weight, based on polymer weight, of a copolymerized monoethylenically unsaturated amino-group containing monomer, based on the weight of the polymer, such as t-butylaminoethyl (meth)acrylate. (iii) The organic polymer phase may further contain 0-2 percent by weight, based on the polymer weight, of a copolymerized multiethylenically unsaturated monomer such as allyl methacrylate, diallyl phthalate, 1,4-butylene glycol dimethacrylate, 1,2-ethylene glycol dimethacrylate, 1,6-hexanediol diacrylate, and divinyl benzene.

In addition, the organic polymer phase may contain 0-5 percent by weight based on the polymer weight, of alternative anionic surfactants such as polymeric surfactants, including polyacrylic gels; copolymerizable surfactants such as hydroxyethyl methacrylate containing 10 moles ethylene oxide units (EO), salts of unsaturated fatty acids such as amine salts of oleic acid, and alpha-olefin sulfonates; and anionic or nonionic copolymerizable surfactants such as compositions sold under the trademark MAXEMUL® 5010, MAXEMUL®5011, MAXEMUL®6106, or MAXEMUL®6112, by Uniqema (ICI Surfactants), Wilmington, Delaware.

Chain transfer agents can be added to the organic polymer phase including halogen compounds such as tetrabromomethane; allyl compounds; or mercaptans such as alkyl thioglycolates, alkyl mercaptoalkanoates. The $C_4$-$C_{22}$ linear or branched alkyl mercaptans may be used to lower the molecular weight of the formed polymer, and/or provide a different molecular weight distribution than would otherwise have been obtained with any free radical generating initiator(s). In such case, linear or branched $C_4$-$C_{22}$ alkyl mercaptans such as n-dodecyl mercaptan and t-dodecyl mercaptan are preferred. Chain transfer agent(s) may be added in one or more additions, continuously, or linearly, and coordinated with the monomer addition, over most or all of the entire reaction period, or during limited portion(s) of the reaction period.

The emulsion may be coagulated and subsequently dried to obtain the silicone organic alloy and/or hybrid polymer without water. The coagulation step can be carried out by various coagulation methods, such as aqueous electrolyte (salt) coagulation using an aqueous solution of a salt of an inorganic acid such as sodium chloride, magnesium acetate, or calcium hypophosphite. It is preferred that the electrolyte solution be prepared with a salt containing a divalent cation such as calcium chloride ($CaCl_2$). Coagulation with a water soluble or partially water soluble solvent such as methanol is also possible. It is preferred to coagulate the first aqueous particle dispersion using aqueous electrolyte coagulation, wherein the aqueous electrolyte solution has a concentration of 0.1-2.0 percent by weight, preferably 0.2-1.0 percent by weight. It is important to control the coagulation temperature, since too high a coagulation temperature results in excessively large particles, causing poor dispersion. In contrast, too low a temperature results in excessively small particles, resulting in a wide particle size span and the generation of excessive dust.

The resulting coagulated slurry is then dried to less than 5 percent by weight of water to form a free flowing powder. Various methods of drying particle slurries are described in Chemical Engineer's Handbook, 5th Ed., Perry and Chilton, Eds. 1973, which relates to the drying of solid-liquid particle dispersions. The preferred drying methods can include fluidized bed dryers, rotary dryers, spray dryers, continuous or batch tray dryers, flash dryers, and pneumatic conveying dryers. During the drying step, it is important to control the drying temperature so that the slurry particles do not fuse among themselves. This can be accomplished by maintaining the temperature of the slurry particles below the $T_g$ of the organic polymer components. If the drying temperature is too high, then the individual polymer particles may fuse together in the powder particles, which may hinder their subsequent dispersion into formulations. A free flowing, low dust powder can be achieved when the water content is less than 5 percent by weight, preferably less than 3 percent by weight, most preferably less than 1 percent by weight.

Some typical ethylenically unsaturated organic monomers useful herein and a simplified depiction of their polymerization are shown below wherein R can represent methyl, ethyl, 2-ethylhexyl, 2-hydroxyethyl, or 2-hydroxypropyl groups.

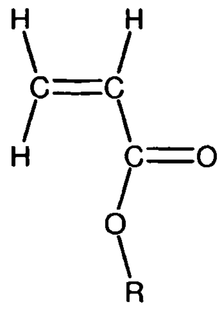
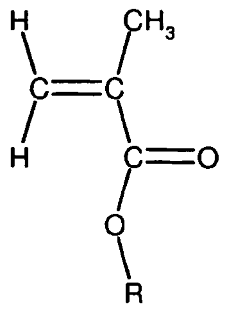
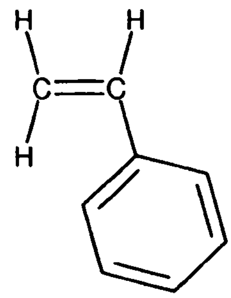

Acrylate Ester Methacrylate Ester Styrene

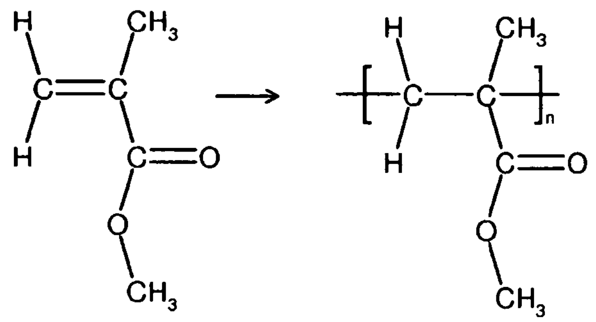

Free Radical Polymerization of Methyl Methacrylate to Poly(methylmethacrylate)

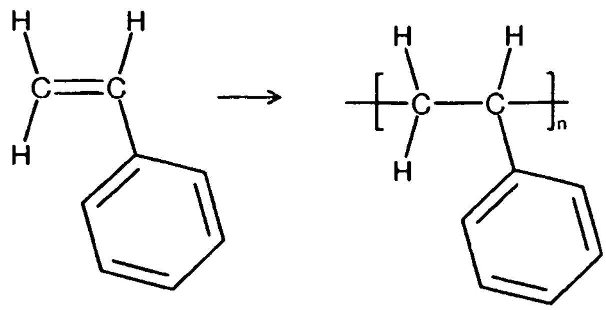

Free Radical Polymerization of Styrene to Polystyrene

Compositions comprising the silicone organic hybrid emulsions may be formulated into personal care products. The personal care compositions of this invention may be in the form of a cream, a gel, a powder, a paste, or a freely pourable liquid. Generally, such compositions can generally be prepared at room temperature if no solid materials at room temperature are presents in the compositions, using simple propeller mixers, Brookfield counter-rotating mixers, or homogenizing mixers. No special equipment or processing conditions are typically required. Depending on the type of form made, the method of preparation will be different, but such methods are well known in the art.

The personal care products may be functional with respect to the portion of the body to which they are applied, cosmetic, therapeutic, or some combination thereof. Conventional examples of such products include, but are not limited to: antiperspirants and deodorants, skin care creams, skin care lotions, moisturizers, facial treatments such as acne or wrinkle removers, personal and facial cleansers, bath oils, perfumes, colognes, sachets, sunscreens, pre-shave and after-shave lotions, shaving soaps, and shaving lathers, hair shampoos, hair conditioners, hair colorants, hair relaxants, hair sprays, mousses, gels, permanents, depilatories, and cuticle coats, make-ups, color cosmetics, foundations, concealers, blushes, lipsticks, eyeliners, mascara, oil removers, color cosmetic removers, and powders, medicament creams, pastes or sprays including antiacne, dental hygienic, antibiotic, healing promotive, nutritive and the like, which may be preventative and/or therapeutic. In general the personal care products may be formulated with a carrier that permits application in any conventional form, including but not limited to liquids, rinses, lotions, creams, pastes, gels, foams, mousses, ointments, sprays, aerosols, soaps, sticks, soft solids, solid gels, and gels. What constitutes a suitable carrier

is readily apparent to one of ordinary skill in the art.

The present compositions can be used in a variety of personal, household, and healthcare applications. In particular, the compositions of the present invention may be used in the personal care products as taught in US Patent Nos. 6,051,216, 5,919,441, 5,981,680; as disclosed in WO 2004/060271 and WO 2004/060101; in sunscreen compositions as taught in WO 2004/060276; in cosmetic compositions also containing film-forming resins, as disclosed in WO 03/105801; in the cosmetic compositions as taught in US Patent Application Publications 2003/0235553, 2003/0072730, 2003/0170188, EP 1,266,647, EP 1,266,648, EP1,266,653, WO 03/105789, WO 2004/000247 and WO 03/106614; as additional agents to those taught in WO 2004/054523; in long wearing cosmetic compositions as taught in US Patent Application Publication 2004/0180032; in transparent or translucent care and/or make up compositions as discussed in WO 2004/054524.

The compositions according to this invention can be used by the standard methods, such as applying them to the human body, e.g. skin or hair, using applicators, brushes, applying by hand, pouring them and/or possibly rubbing or massaging the composition onto or into the body. Removal methods, for example for color cosmetics are also well known standard methods, including washing, wiping, peeling and the like. For use on the skin, the compositions according to the present invention may be used in a conventional manner for example for conditioning the skin. An effective amount of the composition for the purpose is applied to the skin. Such effective amounts generally range from about 1mg/cm$^2$ to about 3 m/cm$^2$. Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The use of the compositions according to the invention on hair may use a conventional manner for conditioning hair. An effective amount of the composition for conditioning hair is applied to the hair. Such effective amounts generally range from about 0.5 g to about 50 g, preferably from about 1 g to about 20 g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit.

Non-limiting examples of additives which may be formulated into the personal care compositions in addition to the silicone organic hybrid emulsions include: additional silicones, anti-oxidants, cleansing agents, colorants, additional conditioning agents, deposition agents, electrolytes, emollients and oils, exfoliating agents, foam boosters, fragrances, humectants, occlusive agents, pediculicides, pH control agents, pigments, preservatives, biocides, other solvents, stabilizers, sun-screening agents, suspending agents, tanning agents, other surfactants, thickeners, vitamins, botanicals, fragrances, waxes, rheology-modifying agents, anti-dandruff, anti-acne, anti-carie and wound healing-promotion agents.

The personal care composition, such as a shampoo or cleanser may contain at least one anionic detersive surfactant. This can be any of the well-known anionic detersive surfactants typically used in shampoo formulations. These anionic detersive surfactants function as cleansing agents and foaming agents in the shampoo compositions of this invention. The anionic detersive surfactants are exemplified by alkali metal sulforicinates, sulfonated glyceryl esters of fatty acids such as sulfonated monoglycerides of coconut oil acids, salts of sulfonated monovalent alcohol esters such as sodium oleylisethianate, amides of amino sulfonic acids such as the sodium salt of oleyl methyl tauride, sulfonated products of fatty acids nitriles such as palmitonitrile sulfonate, sulfonated aromatic hydrocarbons such as sodium alpha-naphthalene monosulfonate, condensation products of naphthalene sulfonic acids with formaldehyde, sodium octahydroanthracene sulfonate, alkali metal alkyl sulfates such as sodium lauryl sulfate, ammonium lauryl sulfate or triethanol amine lauryl sulfate, ether sulfates having alkyl groups of 8 or more carbon atoms such as sodium lauryl ether sulfate, ammonium lauryl ether sulfate, sodium alkyl aryl ether sulfates, and ammonium alkyl aryl ether sulfates, alkylarylsulfonates having 1 or more alkyl groups of 8 or more carbon atoms, alkylbenzenesulfonic acid alkali metal salts exemplified by hexylbenzenesulfonic acid sodium salt, octylbenzenesulfonic acid sodium salt, decylbenzenesulfonic acid sodium salt, dodecylbenzenesulfonic acid sodium salt, cetylbenzenesulfonic acid sodium salt, and myristylbenzenesulfonic acid sodium salt, sulfuric esters of polyoxyethylene alkyl ether including CH3(CH2)6CH2O(C2H4O)2SO3H, CH3(CH2)7CH2O(C2H4O)3.5SO3H, CH3(CH2)8CH2O(C2H40)8SO3H, CH3(CH2)19CH2O(C2H4O)4SO3H, and CH3(CH2) 10CH2O(C2H4O)6SO3H, sodium salts, potassium salts, and amine salts of alkylnaphthylsulfonic acid. Preferably the detersive surfactant is selected from the group consisting of sodium lauryl sulfate, ammonium lauryl sulfate, triethanolamine lauryl sulfate, sodium lauryl ether sulfate, and ammonium lauryl ether sulfate. The anionic detersive surfactant is present in the shampoo compositions of this invention in an amount from about 5 to 50 wt% and preferably about 5 to 25 wt% based on the total weight of the composition.

The personal care composition may contain at least one cationic deposition aid, preferably a cationic deposition polymer. The cationic deposition aid will generally be present at levels of from 0.001 to 5%, preferably from about 0.01 to 1%, more preferably from about 0.02% to about 0.5% by weight. The polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic

nitrogen containing groups such as quaternary ammonium or protonated amino groups, or a mixture thereof. The cationic charge density has been found to need to be at least 0.1 meq/g, preferably above 0.8 or higher. The cationic charge density should not exceed 4 meq/g, it is preferably less than 3 and more preferably less than 2 meq/g. The charge density can be measured using the Kjeldahl method and should be within the above limits at the desired pH of use, which will in general be from about 3 to 9 and preferably between 4 and 8. The cationic nitrogen-containing group will generally be present as a substituent on a fraction of the total monomer units of the cationic deposition polymer. Thus when the polymer is not a homopolymer it can contain spacer noncationic monomer units. Such polymers are described in the CTFA Cosmetic Ingredient Directory, 3rd edition. Suitable cationic deposition aids include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyil substituted monomers preferably have C1-C7 alkyl groups, more preferably C1-3 alkyl groups. Other suitable spacers include vinyl esters, vinyl alcohol, maleic anhydride, propylene glycol and ethylene glycol. The cationic amines can be primary, secondary or tertiary amines, depending upon the particular species and the pH of the composition. In general secondary and tertiary amines, especially tertiary, a-re preferred. Amine substituted vinyl monomers and amines can be polymerized in the amine form and then converted to ammonium by quatemization. Suitable cationic amino and quaternary ammonium monomers include, for example, vinyl compounds substituted with dialkyl aminoalkyl acrylate, dialkylamino alkylmethacrylate, monoalkylaminoalkyl acrylate, monoalkylaminoalkyl methacrylate, trialkyl methacryloxyalkyl ammonium salt, triaikyi acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidine, e.g., alkyl vinyl imidazolium, and quaternized pyrrolidine, e.g., alkyl vinyl imidazolium, alkyl vinyl pyridinium, alkyl vinyl pyrrolidine salts. The alkyl portions of these monomers are preferably lower alkyls such as the C,-C., alkyls, more preferably C, and C2 alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C,-C,hydrocarbyls, more preferably C,-C,, alkyls. The cationic deposition aids can comprise mixtures of monomer units derived from amine- and/or quaternary ammonium-substituted monomer and/or compatible spacer monomers. Suitable cationic deposition aids include, for example: copolymers of 1-vinyl-2-pyrrolidine and 1-vinyl-3-methylimidazolium salt (e.g., Chloride salt) (referred to in the industry by the Cosmetic, Toiletry, and Fragrance Association, "CTFA". as Polyquaternium-16) such as those commercially available from BASF Wyandotte Corp. (Parsippany, NJ, USA) under the LUVIQUAT tradename (e.g., LUVIQUAT FC 370); copolymers of 1-vinyl-2-pyrrolidine and dimethylaminoethyl methacrylate (referred to in the industry by CTFA as Polyquaternium-11) such as those commercially from Gar Corporation (Wayne, NJ, USA) under the GAFQUAT tradename (e.g., GAFQUAT 755N); cationic diallyl quaternary ammonium-containing polymer including, for example, dimethyldiallyammonium chloride homopolymer and copolymers of acrylamide and dimethyl diallyammonium chloride, referred to in the industry (CTFA) as Polyquaternium 6 and Polyquaternium 7, respectively; mineral acid salts of aminoalkyl esters of homo-and co-polymers of unsaturated carboxylic acids having from 3 to 5 carbon atoms, as described in U.S. Patent 4,009,256; and cationic polyacrylamides as described in our copending UK Application No. 9403156.4 (W095/22311). Other cationic deposition aids that can be used include polysaccharide polymers, such as cationic cellulose derivatives and cationic starch derivatives. Cationic polysaccharide polymer materials suitable for use in compositions of the invention include those of the formula: A-O(R-$N^+R^1R^2R^3X^-$) wherein: A is an anhydroglucose residual group, such as starch or cellulose anhydroglucose residual, R is an alkylene oxyalklene, polyoxyalkylene, or hydroxyalkylene group, or combination thereof, R', R~' and R3 independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R', R 2 and R') preferably being about 20 or less, and X is an anionic counterion, as previously described. Cationic cellulose is available from Amerchol Corp. (Edison, NJ, USA) in their Polymer iR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200. Other cationic deposition aids that can be used include cationic guar gum derivatives, such as guar hydroxypropyltrimonium chloride (Commercially available from Celanese Corp. in their Jaguar trademark series). Other materials include quaternary nitrogen-containing cellulose ethers (e.g., as described in U.S. Patent 3,962,418), and copolymers of etherified cellulose and starch (e.g., as described in U.S. Patent 3,958,581).

The personal care composition may contain a foam boosting agent. A foam booster is an agent which increases the amount of foam available from a system at a constant molar concentration of surfactant, in contrast to a foam stabilizer which delays the collapse of a foam. Foam building is provided by adding to the aqueous media, a foam boosting effective amount of a foam boosting. The foam boosting agent is preferably selected from the group consisting of fatty acid alkanolamides and amine oxides. The fatty acid alkanolamides are exemplified by isostearic acid diethanolamide, lauric acid diethanolamide, capric acid diethanolamide, coconut fatty acid diethanolamide, linoleic acid diethanolamide, myristic

acid diethanolamide, oleic acid diethanolamide, stearic acid diethanolamide, coconut fatty acid monoethanolamide, oleic acid monoisopropanolamide, and lauric acid monoisopropanolamide. The amine oxides are exemplified by N-cocodimethylamine oxide, N-lauryl dimethylamine oxide, N-myristyl dimethylamine oxide, N-stearyl dimethylamine oxide, N-cocamidopropyl dimethylamine oxide, N-tallowamidopropyl dimethylamine oxide, bis(2-hydroxyethyl) C12-15 alkoxypropylamine oxide. Preferably a foam booster is selected from the group consisting of lauric acid diethanolamide, N-lauryl dimethylamine oxide, coconut acid diethanolamide, myristic acid diethanolamide, and oleic acid diethanolamide. The foam boosting agent is preferably present in the shampoo compositions of this invention in an amount from about 1 to 15 wt% and more preferably about 2 to 10 wt% based on the total weight of the composition. The composition may further comprise a polyalkylene glycol to improve lather performance. Concentration of the polyalkylene glycol in the shampoo composition may range from about 0.01% to about 5%, preferably from about 0.05% to about 3%, and more preferably from about 0. 1% to about 2%, by weight of the composition. The optional polyalkylene glycols are characterized by the general formula: H(OCH2CHR)n-OH wherein R is selected from the group consisting of H, methyl, and mixtures thereof. When R is H, these materials are polymers of ethylene oxide, which are also known as polyethylene oxides, polyoxyethylenes, and polyethylene glycols. When R is methyl, these materials are polymers of propylene oxide, which are also known as polypropylene oxides, polyoxypropylenes, and polypropylene glycols. When R is methyl, it is also understood that various positional isomers of the resulting polymers can exist. In the above structure, n has an average value of from about 1500 to about 25,000, preferably from about 2500 to about 20,000, and more preferably from about 3500 to about 15,000. Polyethylene glycol polymers useful herein are PEG-2M wherein R equals H and n has an average value of about 2,000 (PEG-2M is also known as Polyox WSR9 N-10, which is available from Union Carbide and as PEG-2,000); PEG-5M wherein R equals H and n has an average value of about 5,000 (PEG-5M is also known as Polyox WSRO N-35 and Polyox WSRS N-80, both available from Union Carbide and as PEG-5,000 and Polyethylene Glycol 300,000); PEG-7M wherein R equals H and n has an average value of about 7,000 (PEG-7M is also known as Polyox WSRO N-750 available from Union Carbide); PEG-9M wherein R equals H and n has an average value of about 9,000 (PEG 9-M is also known as Polyox WSRS N-3333 available from Union Carbide); and PEG 14 M wherein R equals H and n has an average value of about 14,000 (PEG-14M is also known as Polyox WSRO N-3000 available from Union Carbide). Other useful polymers include the polypropylene glycols and mixed polyethylene/polypropylene glycols.

The personal care composition may contain a suspending agent at concentrations effective for suspending the preferred silicone conditioning agent, or other water-insoluble material, in dispersed form in the shampoo compositions. Such concentrations range from about 0.1% to about 10%, preferably from about 0.3% to about 5.0%, by weight of the shampoo compositions. Suspending agents include crystalline suspending agents which can be categorized as acyl derivatives, long chain amine oxides, and mixtures thereof, concentrations of which range from about 0.1% to about 5.0%, preferably from about 0.5% to about 3.0%, by weight of the shampoo compositions. These suspending agents are described in U.S. Patent 4.741,855, which description is incorporated herein by reference. These preferred suspending agents include ethylene glycol esters of fatty acids preferably having from about 16 to about 22 carbon atoms. More preferred are the ethylene glycol stearates, both mono and distearate, but particularly the distearate containing less than about 7% of the mono stearate. Other suitable suspending agents include alkanol amides of fatty acids, preferably having from about 16 to about 22 carbon atoms, more preferably about 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); glyceryl esters (e.g., glyceryl distearate) and long chain esters of long chain alkanol amides (e.g., stearamide diethanolamide distearate, stearamide monoethanolamide stearate). Long chain acyl derivatives, ethylene glycol esters of long chain carboxylic acids, long chain amine oxides, and alkanol amides of long chain carboxylic acids in addition to the preferred materials listed above may be used as suspending agents. For example, it is contemplated that suspending agents with long chain hydrocarbyls having C8-C22 chains may be used. Other long chain acyl derivatives suitable for use as suspending agents include N,N-dihydrocarbyl amido benzoic acid and soluble salts thereof (e.g., Na, K), particularly N,N-di(hydrogenated) C16, C18 and tallow amido benzoic acid species of this family, which are commercially available from Stepan Company (Northfield, Illinois, USA). Examples of suitable long chain amine oxides for use as suspending agents include alkyl (C16-C22) dimethyl amine oxides, e.g., stearyl dimethyl amine oxide. Other suitable suspending agents include xanthan gum at concentrations ranging from about 0.3% to about 3%, preferably from about 0.4% to about 1.2%, by weight of the shampoo compositions. The use of xanthan gum as a suspending agent in silicone containing shampoo compositions is described, for example, in U.S. Patent 4,788,006, which description is incorporated herein by reference. Combinations of long chain acyl derivatives and xanthan gum may also be used as a suspending agent in the shampoo compositions. Such combinations are described in U.S. Patent 4,704,272, which description is incorporated herein by reference. Other suitable suspending agents include carboxyvinyl polymers. Preferred among these polymers are the copolymers of acrylic acid crosslinked with polyallylsucrose as described in U.S. Patent 2,798,053, which description is incorporated herein by reference. Examples of these polymers include Carbopol 934, 940, 941, and 956. available from B. F. Goodrich Company. Other suitable suspending agents include primary amines having a fatty alkyl moiety having at least about 16 carbon atoms, examples of which include

palmitamine or stearamine, and secondary amines having two fatty alkyl moieties each having at least about 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer. Other suitable suspending agents may be used in the shampoo compositions, including those that can impart a gel-like viscosity to the composition, such as water soluble or colloidally water soluble polymers like cellulose ethers (e.g., methylcellulose, hydroxybutyl methylcellulose, hyroxypropylcellulose, hydroxypropyl methylcellulose, hydroxyethyl ethylcellulose and hydroxyethylcellulose), guar gum, polyvinyl alcohol, polyvinyl pyrolidone, hydroxypropyl guar gum, starch and starch derivatives, and other thickeners, viscosity modifiers, gelling agents, etc.

The personal care composition may contain one or more water-soluble emollients including, but not limited to, lower molecular weight aliphatic diols such as propylene glycol and butylene glycol; polyols such as alycerine and sorbitol; and polyoxyethylene polymers such as polyethylene glycol 200. The specific type and amount of water soluble emollient(s) employed will vary depending on the desired aesthetic characteristics of the composition, and is readily determined by one skilled in the art.

The personal care composition may contain various oils. The term "oil" as used herein refers to any material which is substantially insoluble in water. When the composition is to be used in a cosmetic or personal care product, the product components must also be cosmetically acceptable or otherwise meet the conditions of the end use product. Suitable oil components include, but are not limited to, natural oils such as coconut oil; hydrocarbons such as mineral oil and hydrogenated polyisobutene; fatty alcohols such as octyldodecanol; esters such as C12 -C15 alkyl benzoate; diesters such as propylene dipelarganate; and triesters, such as glyceryl trioctanoate and silicones especially cyclomethicone and dimethicone and mixtures thereof. The composition of the invention also contains oils, preferably a mixture of low viscosity and high viscosity oils. Suitable low viscosity oils have a viscosity of 5 to 100 mPa.s at 25°C, and are generally esters having the structure RCO-OR' wherein RCO represents the carboxylic acid radical and wherein OR' is an alcohol residue. Examples of these low viscosity oils include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco-dicaprylate/caprate, decyl isostearate, isodecyl oleate, isodecyl neopentanoate, isohexyl neopentanoate, octyl palmitate, dioctyl malate, tridecyl octanoate, myristyl myristate, octododecanol, or mixtures of octyldodecanol, acetylated lanolin alcohol, cetyl acetate, isododecanol, polyglyceryl-3-diisostearate, or mixtures thereof. The high viscosity surface oils generally have a viscosity of 200-1,000,000 mPa.s at 25°C, preferably a viscosity of 100,000-250,000 mPa.s. Surface oils include castor oil, lanolin and lanolin derivatives, triisocetyl citrate, sorbitan sesquioleate, C10-18 triglycerides, caprylic/capric/triglycerides, coconut oil, corn oil, cottonseed oil, glyceryl triacetyl hydroxystearate, glyceryl triacetyl ricinoleate, glyceryl trioctanoate, hydrogenated castor oil, linseed oil, mink oil, olive oil, palm oil, illipe butter, rapeseed oil, soybean oil, sunflower seed oil, tallow, tricaprin, trihydroxystearin, triisostearin, trilaurin, trilinolein, trimyristin, triolein, tripalmitin, tristearin, walnut oil, wheat germ oil, cholesterol, or mixtures thereof. The suggested ratio of low viscosity to high viscosity oils in the oil phase is 1:15 to 15:1, preferably 1:10 to 10:1 respectively. The preferred formulation of the invention comprises 1 to 20% of a mixture of low viscosity and high viscosity surface oils. Mention may be made, among the optional other non-silicone fatty substances, of mineral oils, such as liquid paraffin or liquid petroleum, of animal oils, such as perhydrosqualene or arara oil, or alternatively of vegatable oils, such as sweet almond, calophyllum, palm, castor, avocado, jojaba, olive or cereal germ oil. It is also possible to use esters of lanolic acid, of oleic acid, of lauric acid, of stearic acid or of myristic acid, for example; alcohols, such as oleyl alcohol, linoleyl or linolenyl alcohol, isostearyl alcohol or octyldodecanol; or acetylglycerides, octanoates, decanoates or ricinoleates of alcohols or of polyalcohols. It is alternatively possible to use hydrogenated oils which are solid at 25°C, such as hydrogenated castor, palm or coconut oils, or hydrogenated tallow; mono-, di-, tri- or sucroglycerides; lanolins; or fatty esters which are solid at 25°C.

The personal care composition may contain various waxes. The waxes or wax-like materials generally have a melting point range of 35 to120°C at atmospheric pressure. Waxes in this category include synthetic wax, ceresin, paraffin, ozokerite, illipe butter, beeswax, carnauba, microcrystalline, lanolin, lanolin derivatives, candelilla, cocoa butter, shellac wax, spermaceti, bran wax, capok wax, sugar cane wax, montan wax, whale wax, bayberry wax, or mixtures thereof. The preferred formulation of the invention comprises about 10-30% of a mixture of waxes. Mention may be made, among the waxes capable of begin used as non-silicone fatty substances, of animal waxes, such as beeswax; vegetable waxes, such as carnauba, candelilla, ouricury or japan wax or cork fibre or sugarcane waxes; mineral waxes, for example paraffin or lignite wax or microcrystalline waxes or ozokerites; synthetic waxes, including polyethylene waxes, and waxes obtained by the Fischer-Tropsch synthesis. Mention may be made, among the silicone waxes, of polymethylsiloxane alkyls, alkoxys and/or esters.

The personal care composition may contain various powders. The powder component of the invention can be generally defined as dry, particulate matter having a particle size of 0.02-50 microns. The particulate matter may be colored or non-colored (for example white). Suitable powders include bismuth oxychloride, titanated mica, fumed silica, spherical silica beads, polymethylmethacrylate beads, micronized teflon, boron nitride, acrylate polymers, aluminum silicate, aluminum starch octenylsuccinate, bentonite, calcium silicate, cellulose, chalk, corn starch, distomaceous earth, fuller's earth, glyceryl starch, hectorite, hydrated silica, kaolin, magnesium aluminum silicate, magnesium carbonate,

magnesium hydroxide, magnesium oxide, magnesium silicate, magnesium trisilicate, maltodextrin, montmorillonite, microcrystalline cellulose, rice starch, silica, talc, mica, titanium dioxide, zinc laurate, zinc myristate, zinc neodecanoate, zinc rosinate, zinc stearate, polyethylene, alumina, attapulgite, calcium carbonate, calcium silicate, dextran, kaolin, nylon, silica silylate, silk powder, serecite, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature. The powder component also comprises various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows, etc. Inorganic pigments generally consist of insoluble metallic salts of certified color additives, referred to as the Lakes or iron oxides.

A pulverulent colouring agent, such as carbon black, chromium or iron oxides, ultramarines, manganese pyrophosphate, iron blue, and titanium dioxide, pearlescent agents, generally used as a mixture with coloured pigments, or some organic dyes, generally used as a mixture with coloured pigments and commonly used in the cosmetics industry, can be added to the composition. In general, these colouring agents can be present in an amount by weight from 0 to 20% with respect to the weight of the final composition.

Pulverulent inorganic or organic fillers can also be added, generally in an amount by weight from 0 to 40% with respect to the weight of the final composition. These pulverulent fillers can be chosen from talc, micas, kaolin, zinc or titanium oxides, calcium or magnesium carbonates, silica, spherical titanium dioxide, glass or ceramic beads, metal soaps derived from carboxylic acids having 8-22 carbon atoms, non-expanded synthetic polymer powders, expanded powders and powders from natural organic compounds, such as cereal starches, which may or may not be crosslinked. The fillers may preferably be present in a proportion of from 0 to 35% of the total weight of the composition, more preferably 5 to 15%. Mention may be made in particular of talc, mica, silica, kaolin, nylon powders (in particular ORGASOL), polyethylene powders, Teflon, starch, boron nitride, copolymer microspheres such as EXPANCEL (Nobel Industrie), polytrap and silicone resin microbeads (TOSPEARL from Toshiba, for example).

The personal care composition may contain sunscreens. These include those which absorb ultraviolet light between about 290-320 nanometers (the UV-B region) such as, but not exclusively, para-aminobenzoic acid derivatives and cinnamates such as octyl methoxycinnamate and those which absorb ultraviolet light in the range of 320-400 nanometers (the UV-A region) such is benzophenones and butyl methoxy dibenzoylmethane. Some additional examples of sunscreen chemicals which may be employed in accordance with the present invention are 2-ethoxyethyl p-methoxycinnamate; menthyl anthranilate; homomenthyl salicylate; glyceryl p-aminobenzoate; isobutyl p-aminobenzoate; isoamyl p-dimethylaminobenzoate; 2-hydroxy-4-methoxybenzophenones sulfonic acid; 2,2'-dihydroxy-4-methoxybenzophenone; 2-hydroxy-4-methoxybenzophenone; 4-mono and 4-bis(3-hydroxy-propyl)amino isomers of ethyl benzoate; and 2-ethylhexyl p-dimethylaminobenzoate. As hydrophilic screening agents which can be used in the invention, mention may be made of those described in Application EP-A-678,292. These hydrophilic screening agents are those containing at least one carboxylic or better still sulphonic acid radical. This acid radical can be in free form or in partially or totally neutralized form. It is possible to use one or more hydrophilic screening agents containing acid functionality. As examples of acidic screening agents containing at least one SO3 H group, mention may be made more particularly of 3-benzylidine-2-camphorsulphonic derivatives. A particularly preferred compound is benzene-1,4-[di(3-methylidenecamphor-10-sulphonic acid)]. This screening agent is a broad-band screening agent capable of absorbing ultraviolet rays with wavelengths of between 280 nm and 400 nm, with absorption maxima of between 320 nm and 400 nm, in particular at about 345 nm. It is used in acid form or salified with a base chosen from triethanolamine, sodium hydroxide and potassium hydroxide. In addition, it can be in cis or trans form. This screening agent is known under the trade name Mexoryl SX. Other specific examples are 4-(3-methylidenecamphor)benzenesulphonic acid, 3-benzylidenecamphor-10-sulphonic acid, 2-methyl-5-(3-methylidenecamphor)benzenesulphonic acid, 2-chloro-5-(3-methylidenecamphor)benzenesulphonic acid, 3-(4-methyl)benzylidenecamphor-10-sulphonic acid, (3-t-butyl-2-hydroxy-5-methyl)benzylidenecamphor-10-sulphonic acid, (3-t-butyl-2-hydroxy-5-methoxy)benzylidenecamphor-10-sulphonic acid, (3,5-di-tert-butyl-4-hydroxy)benzylidenecamphor-10-sulphonic acid, 2-methoxy-5-(3-methylidenecamphor)benzenesulphonic acid, 3-(4,5-methylenedioxy)benzylidenecamphor-10-sulphonic acid, 3-(4-methoxy)benzylidenecamphor-10-sulphonic acid, 3-(4,5-dimethoxy)benzylidenecamphor-10-sulphonic acid, 3-(4-n-butoxy)benzylidenecamphor-10-sulphonic acid, 3-(4-n-butoxy-5-methoxy)benzylidenecamphor-10-sulphonic acid, 2-[4-(camphormethylidene)phenyl]benzimidazole-5-sulphonic acid. Suitable compounds are described in U.S. Pat. No. 4,585,597, patent applications FR 2,236,515, 2,282,426, 2,645,148, 2,430,938 and 2,592,380. The screening agent containing a sulphonic group can also be a sulphonic derivative of benzophenone or 2-phenylbenzimidazole-5-sulphonic acid, having excellent photoprotective power in the UV-B radiation range and is sold under the trade name "Eusolex 232" by the company Merck, benzene-1,4-di(benzimidazol-2-yl-5-sulphonic acid), benzene-1,4-di(benzoxazol-2-yl-5-sulphonic acid). The hydrophilic screening agent(s) can be present in the final composition according to the invention in a content which can range from 0.1 to 20%, preferably from 0.2 to 10%, by weight relative to the total weight of the composition.

As lipophilic screening agents which can be used in the invention, mention may be made advantageously of

the family of screening agents derived from dibenzoylmethane and more especially 4-tert-butyl-4'-methoxydibenzoylmethane, which effectively have a high intrinsic power of absorption. These dibenzoylmethane derivatives, which are products that are well known per se as UV-A active screening agents, are described in particular in French patent applications FR-A-2,326,405 and FR-A-2,440,933, as well as in European patent application EP-A-0,114,607; 4-(tert-butyl)-4'-methoxydibenzoylmethane is moreover currently sold under the trade name "Parsol 1789" by the company Givaudan. Another dibenzoylmethane derivative which is preferred according to the present invention is 4-isopropyldibenzoylmethane, this screening agent being sold under the name "Eusolex 8020" by the company Merck. Similarly octocrylene, a liquid lipophilic screening agent that is already known for its activity in the UV-B range is commercially available, and is sold in particular under the name "Uvinul N 539" by the company BASF. As another lipophilic (or liposoluble) screening agent which can be used in the invention, mention may also be made of p-methylbenzylidenecamphor, which is also known as a UV-B absorber and is sold in particular under the trade name "Eusolex 6300" by the company Merck. The lipophilic screening agent(s) can be present in the composition according to the invention in a content which can range from 0.5 to 30%, preferably from 0.5 to 20%, of the total weight of the composition. Other examples of lipophilic or hydrophilic organic screening agents are given in particular in patent application EP-A-0,487,404. The cosmetic and/or dermatological compositions according to the invention can also contain pigments or alternatively nanopigments (average primary particle size: generally between 5 nm and 100 nm, preferably between 10 and 50 nm) of coated or uncoated metal oxides, such as, for example, nanopigments of titanium oxide (amorphous or crystallized in rutile and/or anatase form), of iron oxide, of zinc oxide, of zirconium oxide or of cerium oxide, which are all photoprotective agents that are well known per se and which act by physically blocking (reflection and/or scattering) UV radiation. Standard coating agents are, moreover, alumina and/or aluminium stearate, and silicones. Such coated or uncoated metal oxide nanopigments are described in particular in patent applications EP-A-0,518,772 and EP-A-0,518,773.

Thickening agent may be added to provide a convenient viscosity. For example, viscosities within the range of 500 to 25,000 $mm^2/s$ at 25°C or more alternatively in the range of 3,000 to 7,000 $mm^2/s$ are usually suitable. Suitable thickening agents are exemplified by sodium alginate, gum arabic, polyoxyethylene, guar gum, hydroxypropyl guar gum, ethoxylated alcohols, such as laureth-4 or polyethylene glycol 400, cellulose derivatives exemplified by methylcellulose, methylhydroxypropylcellulose, hydroxypropylcellulose, polypropylhydroxyethylcellulose, starch, and starch derivatives exemplified by hydroxyethylamylose and starch amylose, locust bean gum, electrolytes exemplified by sodium chloride and ammonium chloride, and saccharides such as fructose and glucose, and derivatives of saccharides such as PEG-120 methyl glucose diolate or mixtures of 2 or more of these. Alternatively the thickening agent is selected from cellulose derivatives, saccharide derivatives, and electrolytes, or from a combination of two or more of the above thickening agents exemplified by a combination of a cellulose derivative and any electrolyte, and a starch derivative and any electrolyte. The thickening agent, where used is present in the shampoo compositions of this invention in an amount sufficient to provide a viscosity in the final shampoo composition of from 500 to 25,000 $mm^2/s$. Alternatively the thickening agent is present in an amount from about 0.05 to 10 wt% and alternatively 0.05 to 5 wt% based on the total weight of the composition.

Stabilizing agents can be used in the water phase of the compositions. Suitable water phase stabilizing agents can include alone or in combination one or more electrolytes, polyols, alcohols such as ethyl alcohol, and hydrocolloids. Typical electrolytes are alkali metal salts and alkaline earth salts, especially the chloride, borate, citrate, and sulfate salts of sodium, potassium, calcium and magnesium, as well as aluminum chlorohydrate, and polyelectrolytes, especially hyaluronic acid and sodium hyaluronate. When the stabilizing agent is, or includes, an electrolyte, it amounts to about 0.1 to 5 wt % and more alternatively 0.5 to 3 wt % of the total composition. The hydrocolloids include gums, such as Xantham gum or Veegum and thickening agents, such as carboxymethyl cellulose. Polyols, such as glycerine, glycols, and sorbitols can also be used. Alternative polyols are glycerine, propylene glycol, sorbitol and butylene glycol. If a large amount of a polyol is used, one need not add the electrolyte. However, it is typical to use a combination of an electrolyte, a polyol and an hydrocolloid to stabilize the water phase, e.g. magnesium sulfate, butylene glycol and Xantham gum.

The silicone emulsions can also be used in anti-perspirant and deodorant compositions under but not limited to the form of sticks, soft solid, roll on, aerosol, and pumpsprays. Some examples of antiperspirant agents and deodorant agents are Aluminum Chloride, Aluminum Zirconium Tetrachlorohydrex GLY, Aluminum Zirconium Tetrachlorohydrex PEG, Aluminum Chlorohydrex, Aluminum Zirconium Tetrachlorohydrex PG, Aluminum Chlorohydrex PEG, Aluminum Zirconium Trichlorohydrate, Aluminum Chlorohydrex PG, Aluminum Zirconium Trichlorohydrex GLY, Hexachlorophene, Benzalkonium Chloride, Aluminum Sesquichlorohydrate, Sodium Bicarbonate, Aluminum Sesquichlorohydrex PEG, ,Chlorophyllin-Copper Complex, Triclosan, Aluminum Zirconium Octachlorohydrate, and Zinc Ricinoleate.

The composition according to the invention can also be under the form of aerosols in combination with propellant gases, such as carbon dioxide, nitrogen, nitrous oxide, volatile hydrocarbons such as butane, isobutane, or propane and chlorinated or fluorinated hydrocarbons such as dichlorodifluoromethane and dichlorotetrafluoroethane or dimethylether.

Silicone compositions other than the silicone-organic alloy/hybrid emulsions, may also be included in the personal care compositions. For example, such silicones include; silicone fluids, gums, resins, elastomers; silicone surfactants and emulsifiers such as silicone polyethers, organofunctional silicones such as aminofunctional silicones and alkylmethylsiloxanes.

Alkylmethylsiloxanes may be included in the present compositions. These siloxane polymers generally will have the formula $Me_3SiO[Me_2SiO]_y[MeRSiO]_zSiMe_3$, in which R is a hydrocarbon group containing 6-30 carbon atoms, Me represents methyl, and the degree of polymerization (DP), i.e., the sum of y and z is 3-50. Both the volatile and liquid species of alkymethysiloxanes can be used in the composition.

Silicone gums may be included in the present compositions. Polydiorganosiloxane gums are known in the art and are available commercially. They consist of generally insoluble polydiorganosiloxanes having a viscosity in excess of 1,000,000 centistoke ($mm^2/s$) at 25 °C., alternatively greater than 5,000,000 centistoke ($mm^2/s$) at 25 °C. These silicone gums are typically sold as compositions already dispersed in a suitable solvent to facilitate their handling. Ultra-high viscosity silicones can also be included as optional ingredients. These ultra-high viscosity silicones typically have a kinematic viscosity greater than 5 million centistoke ($mm^2/s$) at 25 °C, to about 20 million centistoke ($mm^2/s$) at 25 °C. Compositions of this type in the form of suspensions are most preferred, and are described for example in US Patent 6.013,682 (January 11, 2000).

Silicone resins may be included in the present compositions. These resin compositions are generally highly crosslinked polymeric siloxanes. Crosslinking is obtained by incorporating trifunctional and/or tetrafunctional silanes with the monofunctional silane and/or difunctional silane monomers used during manufacture. The degree of crosslinking required to obtain a suitable silicone resin will vary according to the specifics of the silane monomer units incorporated during manufacture of the silicone resin. In general, any silicone having a sufficient level of trifunctional and tetrafunctional siloxane monomer units, and hence possessing sufficient levels of crosslinking to dry down to a rigid or a hard film can be considered to be suitable for use as the silicone resin. Commercially available silicone resins suitable for applications herein are generally supplied in an unhardened form in low viscosity volatile or nonvolatile silicone fluids. The silicone resins should be incorporated into compositions of the invention in their non-hardened forms rather than as hardened resinous structures.

Silicone carbinol Fluids may be included in the present compositions. These materials are described in WO 03/101412 A2, and can be commonly described as substituted hydrocarbyl functional siloxane fluids or resins.

Water soluble or water dispersible silicone polyether compositions may be included in the present compositions: These are also known as polyalkylene oxide silicone copolymers, silicone poly(oxyalkylene) copolymers, silicone glycol copolymers, or silicone surfactants. These can be linear rake or graft type materials, or ABA type where the B is the siloxane polymer block, and the A is the poly(oxyalkylene) group. The poly(oxyalkylene) group can consist of polyethylene oxide, polypropylene oxide, or mixed polyethylene oxide/polypropylene oxide groups. Other oxides, such as butylene oxide or phenylene oxide are also possible.

Compositions according to the invention can be used in w/o, w/s, or multiple phase emulsions using silicone emulsifiers. Typically the water-in-silicone emulsifier in such formulation is non-ionic and is selected from polyoxyalkylene-substituted silicones, silicone alkanolamides, silicone esters and silicone glycosides. Suitable silicone-based surfactants are well known in the art, and have been described for example in US 4,122,029 (Gee et al.), US 5.387,417 (Rentsch), and US 5,811,487 (Schulz et al).

## Examples

These examples are intended to illustrate the invention to one of ordinary skill in the art and should not be interpreted as limiting the scope of the invention set forth in the claims. All measurements and experiments were conducted at 23°C, unless indicated otherwise.

Examples 1-5 below are representative of the procedures used to prepare the emulsions shown in Table 1. Typically, the molecular weight, Mw, of the silicone phase ranges from 4,000 to 300,000 g/mole and the organic phase from 300,000 to 2 million.

### Reference Example 1

*Preparation of a Silicone Emulsion by Emulsion Polymerization*

To a one liter glass reactor was added 390.0 gram of water, 23.9 gram of nonionic surfactant polyoxyethylene (23) lauryl ether, and 23.6 gram of dodecylbenzenesulfonic acid. The mixture was heated to 95 °C with mixing. When the temperature reached 95°C, 273.2 grams of octamethylcyclotetrasiloxane (D4) was added and allowed to react for 3 hours. The emulsion was cooled to 30°C and stirred for an additional 3 hours. Lower or higher viscosity silicone emulsions may be obtained by increasing or reducing the temperature, respectively, and by reducing or increasing the holding time at that temperature. The emulsion was neutralized with 23.30 grams of a 50 percent aqueous solution of triethanolamine (TEA) and allowed to cool to room temperature with stirring. The resulting silicone emulsion had a non-volatile content of 44.3 percent, a particle size of 85 nanometer (0.085 micrometer), and an internal silicone phase viscosity of 167,000 centipoise (mPa s).

Reference Example 2

*Preparation of a Silicone-Poly (Butyl Acrylate) 50-50 Emulsion using a Batch Process*

A one liter jacketed reactor was charged with 250.1 gram of a silicone emulsion having essentially the same composition as the silicone emulsion from Example 1. It had a particle size of 85 nanometer (0.085 micrometer), an internal phase viscosity of 167,000 cP (mPa s), and a non-volatile content of 44.3 percent. With continuous mixing, 379.2 gram of deionized water, 110.8 gram of butyl acrylate, and 0.6 gram of a VAZO@) initiator were added to the silicone emulsion, and mixed for 4 hours at room temperature. The temperature of the reactor was brought to 75°C to initiate polymerization, held at that temperature for 2 hours, and then cooled to room temperature with stirring. The final emulsion had a non-volatile content of 30.0 percent and a particle size of 95 nanometers (0.095 micrometer).

Reference Example 3

*Preparation of a Silicone-Poly (Methyl Acrylate/ Veova™ 10) Emulsion Using a Semi-Continuous Process*

A one liter reactor was charged with 363.2 grams of the silicone emulsion having essentially the same composition as the silicone emulsion from Example 1. The mixture was heated to 85°C with mixing. When the temperature reached 85 °C, separate monomer and aqueous feeds were started, using liquid metering pumps. The monomer feed consisted of 76.6 grams of methyl acrylate and 76.6 grams of Veova™ 10 (Veova is a trademark of Resolution Performance Products) which were added over 2 hours. The aqueous feed was added over 2.5 hours and consisted of 205.7 gram of deionized water, 0.5 gram of sodium persulfate, and 0.3 grams of sodium bicarbonate. The emulsion was allowed to cool to room temperature with stirring. The emulsion may be stripped using a rotary evaporator to remove residual acrylate monomer. The final emulsion had a non-volatile content of 42.2 percent and a particle size of 78 nanometers (0.078 micrometer).

Example 4

*Preparation of an Amino-functional silicone Emulsion by Emulsion Polymerization*

To a 1 liter, 3-necked round bottom flask were added 283.50 g of water, 19.60 g of RENEX 30 (tridecyl poly-12-oxyethylene) and 12.81 g of Arquad 16-29 (cetyltrimethylammonium chloride). The contents of the flask were stirred to dissolve the surfactants. Stirring continued as 245 g of a mixture of cyclosiloxanes comprising approximately 96 wt% octamethylcyclotetrasiloxane, 4 wt% decamethylcyclopentasiloxane and a trace of hexamethylcyclotrisiloxane was added to the flask and heated to 90°C. When the temperature was stable, 0.25% of aminopropyltrimethoxysilane and 2.8 g of 50% NaOH were added. The flask was held at 90° C. with stirring for 6 hours. The reaction was neutralized by adding 2.31 g of acetic acid diluted in 14.84 g of water. When the temperature had cooled below 50° C., 107.38 g of water and 0.21 g of KATHON GC (biocide) were added. The particle size was 136 nm.

Example 5

*Preparation of an Aminosilicone-Poly (Methyl Acrylate/Methyl Methacrylate/Vinyl Acetate) Emulsion Using a Batch Process*

A one liter reactor was charged with 319.8 grams of an aminosilicone emulsion having essentially the same composition as the aminosilicone emulsion from Example 4. It had a particle size of 136 nanometers (0.136 micrometer), an internal phase viscosity of 4000 cP (mPa s), and a non-volatile content of 35.0 percent. With continuous mixing, 215.0 grams of deionized water, 67.9 grams of methyl acrylate, 33.6 grams of methyl methacrylate, 33.7 grams of vinyl acetate, and 0.7 gram of a VAZO@) initiator were added to the silicone emulsion, and mixed for 4 hours at room temperature. The temperature of the reactor was brought to 85 °C to initiate polymerization, held at that temperature for 2 hours, and then cooled to room temperature with stirring. The final emulsion had a non-volatile content of 37.0 percent and a particle size of 150 nanometers (0.150 micrometer).

*Table 1 - Emulsion Compositions*

| Emulsion ID | Emulsion Composition Silicone//organic (wt//wt) | Type of surfactant | Particle Size (nm) | % non-volatile content |
|---|---|---|---|---|
| A | Aminosilicone// poly(methyl acrylate/ methyl methacrylate/ vinyl acetate) (50:50) | Cationic | 150 | 24.2 |
| B | Aminosilicone// poly(butyl acrylate/ methyl methacrylate) (30:70) | Cationic | 170 | 22.8 |
| C | Aminosilicone//poly(methyl methacrylate/ butyl acrylate) (30:70) | Cationic | 169 | 30.1 |
| D | Aminosilicone//poly(methyl acrylate/ methyl methacrylate/ vinyl acetate) (30:70) | Cationic | 170 | 30.3 |
| E | Silicone//poly(methyl acrylate) (50:50) | Anionic | 82 | 41.4 |
| F | Silicone//poly(ethyl acrylate) (50:50) | Anionic | 77 | 47.3 |
| G | Silicone//poly(methyl acrylate/Veova 10) (50:50) | Anionic | 78 | 35.5 |
| H | Silicone//poly(butyl acrylate) (50:50) | Anionic | 95 | 30.1 |
| I | Silicone//poly(butyl acrylate/methyl methacrylate/polyethylene glycol diacrylate) (11:89) | Anionic | 61 | 39.5 |

*Example 6 - Hair Conditioner Formulations*

Samples of silicone acrylate emulsions were added to rinse-off conditioning formulations using two percent by weight of the silicone acrylate. The conditioning formulations are shown in Table 1. The conditioners of the present invention were prepared using A, B and H silicone acrylate containing emulsions. A conditioner containing a commercial amino siloxane emulsion which provides good conditioning properties was also tested for comparison purposes.

*Table 2 - Conditioners*

| Ingredient | Silicone Acrylate Emulsion (Weight Percent) | Silicone Acrylate Emulsion (Weight Percent) | Silicone Acrylate Emulsion (Weight Percent) | Amino Siloxane Emulsion(Weight Percent) |
|---|---|---|---|---|
| Deionized Water | q.s. to 100 % | q.s. to 100% | q.s. to 100 % | q.s. to 100 % |
| Hydroxyethylcellulose[1] | 1.5 | 1.5 | 1.5 | 1.5 |
| Cetearyl Alcohol[2] | 1.0 | 1.0 | 1.0 | 1.0 |
| PEG-100 Stearate & Glyceryl Stearate[3] | 1.0 | 1.0 | 1.0 | 1.0 |
| A Emulsion[4] | 8.3 | ---- | ---- | ---- |
| B Emulsion[5] | ---- | 8.8 | ---- | ---- |
| H Emulsion[6] | ---- | ---- | 7.3 | ---- |
| Amino Siloxane Emulsion[7] | ---- | ---- | ---- | 5.7 |
| DMDM Hydantoin[8] | 0.2 | 0.2 | 0.2 | 0.2 |

1. Natrosol® 250 HHR available from Hercules of Wilmington. DE
2. Crodocol CS-50® available from Croda Inc. of Edison, NJ
3. Arlacel® 165 available from Uniqema of Wilmington. DE
4. A, concentration based on 2% active silicone acrylate level, (24.2% Active)
5. B, concentration based on 2% active silicone acrylate level, (22.8% Active)
6. H, concentration based on 2% active silicone acrylale level, (27.6% Active)
7. Dow Corning® 949 Emulsion available from Dow Corning. Midland, MI, concentration based on 2% active silicone level (35% active)
8. Glydant® available from Lonza. Inc. of Fairlawn, NJ

Deionized water is added to the mixing vessel and heated to 75°C. With moderate agitation, the hydroxyethyl cellulose is dispersed until fully dissolved. Heat is decreased to 60°C and cetearyl alcohol and PEG-100 stearate and glyceryl stearate is added. Heat is then decreased to 40° and the silicone silicone acrylate emulsion is added to the base conditioner. The conditioner is mixed for 5-10 minutes and then DMDM hydantoin is added. The water loss is compensated for and the formulation is mixed for an additional 5 minutes. The final pH of the conditioner formulations are all approximately 6-7.

*Procedure - Preparation of Hair Sample*

Slightly bleached European human hair from International Hair Importer and Products, Inc., was used for testing the conditioners prepared herein. A master hand of hair about eight inches in length was subdivided into a series of individual hair tresses. Each tress weighed about 2.5 grams. A 0.5 inch (1.27 cm) of the root end of the hair was trimmed and glued to a 2 inch by 2 inch (5.08 cm by 5.08 cm) plastic tab using DUCO CEMENT ®. The cement was allowed to dry, and the hair tress was combed and trimmed to a length such that six inches (15.24 cm) of hair extended below the bottom of the plastic tab. A hole was punched in middle of tab about one fourth inch (0.635 cm) from its top. Each tress was rinsed for 15 seconds under a stream of 40 °C tap water. Using a pipette, 1.0 gram of a solution containing nine percent of sodium lauryl sulfate was applied and lathered through the tress for 30 seconds. The tress was rinsed for 30 seconds under running water. Excess water was removed from the tress by passing the tress between the index and middle fingers of the hand. The tresses were placed on a tray covered with paper towels and dried overnight. Each tress was hand combed three times with the narrow teeth of an ACE ® comb, and evaluated using INSTRON WET and INSTRON DRY COMBING procedures.

INSTRON procedures are standard, recognized, and industrially acceptable protocols, see for example, US Patents 5,389,364 (February 14, 1995), 5,409,695 (April 25, 1995), 5,419,627 (May 30, 1995), and 5,504,149 (April 2, 1996).

For tests involving rinse-off conditioners, hair tresses are rinsed with tap water for 30 seconds at 40°C. The test conditioner is applied to the tress in the amount of 0.8 gram, and the tress is stroked for 30 seconds. The tress is rinsed for 30 seconds under tap water at 40°C. Excess water is removed by pulling the tress through the index and middle fingers of the hand. The tresses are allowed to dry separately on a paper towel overnight at room temperature. The tresses are combed once before performing an INSTRON study.

***Test Procedure***

INSTRON COMBING is an industry recognized test for determining hair conditioning by the ease of wet combing and the ease of dry combing. The test employs an INSTRON strain gauge, which is equipped to measure the force required to comb the hair. The conditioning performance is based on the ability of a particular hair treatment formulation, such as a shampoo or a hair conditioner, to reduce the force required to comb the hair with the INSTRON strain gauge. The force is reported as an Average Combing Load (ACL). The lower the number of the ACL value, the better is the conditioning effect imparted by the formulation being tested. Typically, ACL baselines are initially established using untreated tresses that have only been washed with a sodium lauryl sulfate solution. The effectiveness of a treatment can then be expressed as an ACL of the treated tress or percent reduction in ACL, calculated using the relationship:

*(untreated hair ACL -treated hair ACL) x 100 divided by the untreated hair ACL*

According to the INSTRON WET COMBING method, hair is first wetted by dipping it into distilled water, and then the hair is detangled by combing the tress three times. The tress is then retangled by dipping in distilled water three times. Excess water is removed by passing the tress through the index and middle fingers of the hand twice. The tress is placed on a hanger and INSTRON combed. Retangling and INSTRON combing are repeated until all data points are collected. An average combing force of three tresses is measured for each treatment.

According to the INSTRON DRY COMBING method, hair is detangled by combing the tress 3 times. Then hair is retangled by swirling the tress clockwise 3 times and swirling it counter clockwise 3 times. The tress is then placed on a hanger and INSTRON combed. Retangle and Instron combing are repeated until all data points are collected. An average combing force for three tresses is measured for each treatment.

The results of INSTRON WET COMBING using Conditioners from Table 2 are shown in Table 3. The results show that the A and H silicone acrylate emulsion containing conditioners provided a reduction in wet combing force. The conditioners containing the silicone acrylate emulsions did not outperform the amino functional silicone emulsion containing conditioner; however, they did well considering that the acrylate:silicone ratio was 50:50 to 70:30. The conditioners containing the silicone acrylate containing emulsions are therefore capable of improving the wet conditioning properties of hair.

The results of INSTRON DRY COMBING tests conducted with the Conditioners from Table 2 are shown in Table 4. Table 4 shows that the silicone acrylate containing emulsions of the present invention provided a significant reduction in dry combing force. Conditioners containing emulsions A and B showed comparable performance to the amino functional silicone emulsion. The conditioners containing the silicone acrylate containing emulsions are therefore capable of significantly improving the dry conditioning properties of hair.

*Table 3 - INSTRON WET COMBING*

| Conditioner | Average Percent Reduction |
|---|---|
| A | 36 |
| B | 12 |
| H | 43 |
| Amino Siloxane Emulsion | 73 |

*Table 4 - INSTRON DRY COMBING*

| Conditioner | Average Percent Reduction |
|---|---|
| A | 75 |
| B | 68 |
| H | 56 |
| Amino Siloxane Emulsion | 74 |

*Example 7 - Hair Conditioner Formulations*

Samples of silicone-acrylate emulsions were added to rinse-off conditioning formulations using two percent by weight of the silicone-acrylate polymer. The conditioning formulations are shown in Table 5. The conditioners of the present invention were prepared using C and D silicone-acrylate containing emulsions. A conditioner containing a commercial amino siloxane emulsion which provides good conditioning properties was also tested for comparison purposes at two different percent silicone active levels. The two percent active silicone level corresponds to the same active level of the silicone-acrylate in the conditioner and the 0.6% active level corresponds to the active silicone level in the silicone acrylate polymer. A conditioner containing a commercial anionic polydimethylsiloxane emulsion was also tested for comparison purposes at the 1.0% active silicone level, which corresponds to the active silicone level in the silicone-acrylate polymer.

*Table 5*

| **Ingredient** | **Silicone Acrylate Emulsion** (Weight Percent) | **Silicone Acrylate Emulsion** (Weight Percent) | **Amino Siloxane Emulsion** (Weight Percent) | **Amino Siloxane Emulsion** (Weight Percent) | **Dimethyl Siloxane Emulsion** (Weight Percent) |
|---|---|---|---|---|---|
| Deionized Water | q.s. to 100 % | q.s. to 100% | q.s. to 100 % | q.s. to 100 % | q.s. to 100 % |
| Hydroxyethylcellulose[1] | 1.5 | 1.5 | 1.5 | 1.5 | 1.5 |
| Cetearyl Alcohol[2] | 1.0 | 1.0 | 1.0 | 1.0 | 1.0 |
| PEG-100 Stearate & Glyceryl Stearate[3] | 1.0 | 1.0 | 1.0 | 1.0 | 1.0 |
| C Emulsion[4] | 6.6 | | | | |
| D Emulsion[5] | | 6.6 | | | |
| Amino Siloxane Emulsion[6] | | | 5.7 | | |

(continued)

| Ingredient | **Silicone Acrylate Emulsion** (Weight Percent) | **Silicone Acrylate Emulsion** (Weight Percent) | **Amino Siloxane Emulsion** (Weight Percent) | **Amino Siloxane Emulsion** (Weight Percent) | **Dimethyl Siloxane Emulsion** (Weight Percent) |
|---|---|---|---|---|---|
| Amino Siloxane Emulsion[7] | | | | 1.7 | |
| Dimethyl Siloxane Emulsion[8] | | | | | 1.7 |
| DMDM Hydantoin[9] | 0.2 | 0.2 | 0.2 | 0.2 | 0.2 |

1. Natrosol® 250 HHR available from Hercules of Wilmington. DE
2. Crodocol CS-50® available from Croda Inc. of Edison. NJ
3. Arlacel® 165 available from Uniqema of Wilmington, DE
4. C, concentration based on 2% active silicone acrylate level, (30.1% Active)
5. D, concentration based on 2% active silicone acrylate level, (30.3% Active)
6. Dow Corning® 949 Emulsion available from Dow Corning, Midland. MI, concentration based on 2% active silicone level (35% active)
7. Dow Corning® 949 Emulsion available from Dow Corning, Midland. MI, concentration based on 0.6% active silicone level (35% active)
8. Dow Corning® 1785 Emulsion available from Dow Corning, Midland, MI, concentration based on 1 % active silicone level (60% active)
9. Glydant® available from Lonza, Inc. of Fairlawn, NJ

Deionized water is added to the mixing vessel and heated to 75°C. With moderate agitation, the hydroxyethyl cellulose is dispersed until fully dissolved. Heat is decreased to 60°C and cetearyl alcohol and PEG-100 stearate and glyceryl stearate is added. Heat is then decreased to 40°C and the silicone-acrylate emulsion is added to the base conditioner. The conditioner is mixed for 5-10 minutes and then DMDM hydantoin is added. The water loss is compensated for and the formulation is mixed for an additional 5 minutes. The final pH of the conditioner formulations are all approximately 6-7.

The results of INSTRON WET COMBING using Conditioners from Table 5 are shown in Table 6. The results show that the conditioners containing C and D silicone-acrylate emulsions provided a small reduction in wet combing force. The conditioners containing the silicone-acrylate emulsions did not outperform the amino functional silicone emulsion containing conditioner; however, they did well considering that the polyacrylate:silicone ratio was 50:50 to 70:30. The conditioners containing the silicone-acrylate containing emulsions are therefore capable of improving the wet conditioning properties of hair.

The results of INSTRON DRY COMBING tests conducted with the Conditioners from Table 5 are shown in Table 7. Table 7 shows that the silicone-acrylate containing emulsions of the present invention provided a significant reduction in dry combing force. Conditioners containing emulsions C and D showed comparable performance to the amino functional silicone emulsion and also demonstrated better performance compared to the dimethyl emulsion. The conditioners containing the silicone-acrylate emulsions are therefore capable of significantly improving the dry conditioning properties of hair.

*Table 6 - INSTRON WET COMBING*

| Conditioner | Average Percent Reduction |
|---|---|
| C | 15.1 |
| D | 18.6 |
| Amino Siloxane Emulsion (2%) | 83.9 |
| Amino Siloxane Emulsion (0.6%) | 34.5 |
| Dimethyl Emulsion (1%) | -20.2 |

*Table 7 - INSTRON DRY COMBING*

| Conditioner | Average Percent Reduction |
|---|---|
| C | 56.0 |
| D | 58.5 |
| Amino Siloxane Emulsion (2%) | 66.4 |
| Amino Siloxane Emulsion (0.6%) | 63.3 |
| Dimethyl Emulsion (1%) | 26.0 |

*Example 8 - Conditioning Shampoo Formulation*

Samples of silicone-acrylate emulsions were added to shampoo formulations using two percent by weight of the silicone-acrylate polymer. The shampoo formulations are shown in Table 8. The shampoos of the present invention were prepared using C and D silicone-acrylate emulsions. A shampoo containing a commercial dimethyl siloxane emulsion which provides good conditioning properties was also tested for comparison purposes.

*Table 8 - Conditioning Shampoos*

| Ingredient | Control (Weight %) | Silicone Acrylate Emulsion (Weight %) | Silicone Acrylate Emulsion (Weight %) | Silicone Acrylate Emulsion (Weight %) | High Molecular Weight Nonionic Emulsion (Weight %) |
|---|---|---|---|---|---|
| Deionized Water | q.s. to 100% | q.s. to 100% | q.s. to 100% | q.s. to 100% | q.s. to 1.00% |
| Sodium Laureth Sulfate[1] | 30 | 30 | 30 | 30 | 30 |
| Cocamide DEA[2] | 3.0 | 3.0 | 3.0 | 3.0 | 3.0 |
| Cocamidopropyl Betaine[3] | 1.5 | 1.5 | 1.5 | 1.5 | 1.5 |
| Polyquaternium-10[4] | 0.3 | 0.3 | 0.3 | 0.3 | 0.3 |
| PEG-150 Pentaerythrityl Tetrastearate[5] | 7.0 | 7.0 | 7.0 | 7.0 | 7.0 |
| C[6] | | 6.6 | | | |
| D[7] | | | 6.6 | | |
| Divinyldimethicone/dimethicone copolymer Emulsion[8] | | | | | 3.3 |
| DMDM Hydantoin[9] | 0.4 | 0.4 | 0.4 | 0.4 | 0.4 |

1. Standapol ES-3® available from Cognix Corp. of Cincinnati. OH
2. Monamid 705® available from Uniqema of New Castle. DE
3. Monateric CAB-LC® available from Uniquema of New Castle. DE
4. UCARE Polymer JR-30M available from Dow/Amerchol of Midland. MI
5. Crothix ® available from Croda Inc. of Edison. NJ
6. C. concentration based on 2% active silicone acrylate level. (30.1% Active)
7. D. concentration based on 2% active silicone acrylate level. (30.3% Active)
8. HMW 2220 Nonionic Emulsion, available from Dow Corning. Midland. M1. concentration based on 2% active silicone level. (60% Active)
9. Glydant ® available from Lonza, Inc. of Fairlawn. NJ

Deionized water is added to the mixing vessel. In order to keep the active silicone loading constant throughout

testing, it is necessary to adjust the water level added depending on the percent active silicone in the various emulsions used. With moderate agitation, the polyquaternium-10 is dispersed until fully dissolved. This is then heated to 75°C and the PEG-150 pentaerythrityl tetrastearate is added with continual mixing. Heat is decreased to 40°C and sodium lauryl ether sulfate, cocamide DEA cocamidopropyl betaine are added in that order. When completely incorporated, silicone acrylate emulsion is added to the base shampoo. The shampoo is mixed for 5-10 minutes and then DMDM hydantoin is added. The water loss is compensated for and the formulation is mixed for an additional 5 minutes. The final pH of the shampoo formulations are approximately 5.5-6.0.

The results of INSTRON WET and DRY COMBING using Shampoos from Table 8 are shown in Tables 9 and 10. The dry combing results show that the silicone-acrylate emulsions in the shampoo formulations provided a significant reduction in combing force, compared to the Control shampoo which contained no silane or siloxane. The dry performance of the shampoo containing the D emulsion was slightly lower compared to the Divinyldimethicone/dimethicone copolymer emulsion; however, it was much improved compared to the control. For wet combing, the silicone-acrylate emulsions provided a reduction in wet combing force. Shampoo formulations containing the silicone-acrylate emulsions are therefore capable of significantly improving the wet and dry conditioning properties of hair.

*Table 9 - INSTRON WET COMBING RESULTS*

| **Shampoo** | **Average % Reduction** |
|---|---|
| Control | -7.9 |
| C | 8.4 |
| D | 16.6 |
| Divinyldimethicone/dimethicone copolymer Emulsion | 56.2 |

*Table 10 - INSTRON DRY COMBING RESULTS*

| **Shampoo** | **Average % Reduction** |
|---|---|
| Control | 9.2 |
| C | 35.0 |
| D | 45.7 |
| Divinyldimethicone/dimethicone copolymer Emulsion | 62.8 |

***<u>Curl Retention</u>***

***<u>Test Procedure</u>***

CURL RETENTION is an industry recognized test for determining hair styling and hold properties by subjecting curled hair tresses to constant temperature and humidity conditions for a specified period of time. Curl retention is measured by recording the difference in length of curled hair tresses before and during high humidity and constant temperature conditions. Already prepared natural virgin brown round hair tresses weighing 2 gram and measuring 25 cm long are employed. To pre-treat all of the tresses, 1.0 gram of a solution containing nine percent of sodium lauryl sulfate is applied and lathered through each tress for 30 seconds. Each tress is rinsed for 30 seconds under running water. Excess water is removed from each tress by passing the tress between the index and middle fingers of the hand. The tresses are placed on a tray covered with paper towels and dried overnight. Each tress is hand combed three times with the narrow teeth of a comb. Each tress is then wet for 15 seconds under tap water at 37 °C and the excess water is removed by pulling the tress through the index and middle fingers of the hand.

Then each of the tresses is treated with either 500 microliters of a 6 % active silicone-acrylate emulsion, 2% active silicone-acrylate emulsion in a mousse formulation or 4% active silicone-acrylate emulsion in a styling gel formulation. Each tress is curled around a 1/4" spiral perm rod and dried in a 40°C oven overnight. The tresses are removed from the rod, keeping the curl intact and hung in a humidity chamber. The conditions of the humidity chamber are 25°C and 70% relative humidity. The tress lengths are then measured periodically over 5 hours. Following the test, the maximum tress length is measured by unrolling it completely. An average of two tresses is measured for each treatment. The percent curl retention is calculated using the relationship:

$$(maximum\ tress\ length - tress\ length\ at\ specific\ time) / (maximum\ tress\ length - tress\ length\ at\ time = 0) \bullet 100$$

*Example 9 - Leave-On Conditioner Application*

Silicone-acrylate emulsions of the present invention were further diluted to a 6% active concentration of silicone-acrylate polymer, and 500 microliters of each of the diluted emulsions was applied to hair tresses for curl retention testing. The curl retention results are shown in Tables 11, 12 and 13.

*Table 11* - Curl Retention - Silicone-Acrylate Emulsion 6% Active Dilution

| Dilution Containing Silicone-Acrylate Emulsion | Curl Retention (%) after 5 hrs | Observations |
|---|---|---|
| Deionized Water | 30 | Difficult to comb, no spring |
| H | 39 | Easy to comb, some spring, no visible residue or coated feel |
| E | 43 | Easy to comb, good curl and spring, some white residue after combing |
| F | 44 | More difficult to comb, tightest curl, white residue |

***Table 12*** **- Curl Retention - Silicone Acrylate Emulsion 6% Active Dilution**

| Dilution Containing Silicone Acrylate Emulsion | Curl Retention (%) after 5 hrs | Observations |
|---|---|---|
| Deionized Water | 40 | Difficult to comb, loose curl, no spring |
| A | 42 | Easy to comb, soft, some spring and curl back |
| B | 47 | Some resistance to combing, soft, some spring, some white visible residue |

***Table 13*** **- Curl Retention - Silicone Acrylate Emulsion 6% Active Dilution**

| Dilution Containing Silicone Acrylate Emulsion | Curl Retention (%) after 5 hrs | Observations |
|---|---|---|
| Deionized Water | 35 | Difficult to comb through ends of hair, minimum spring |
| C | 49 | Easy to comb, smooth feel, some spring and curl back |
| D | 50 | Easy to comb, soft/smooth feel, good curl definition, some spring and good curl back |
| PVP/VA | 53 | Difficult to comb hair, especially ends, not noticeably smooth, some spring |

The results in Tables 11, 12 and 13 demonstrate that the silicone-acrylate emulsions provide flexible hold and styling benefits, in addition to imparting a nice soft feel to the hair. Even when the tresses were stretched or combed to remove the curl, they were observed to bounce right back to their original shaped curl. These results show that the curl retention properties are similar to those obtained using a commercial organic based styling resin; however, conditioning properties such as combing and feel are significantly better when using the silicone-acrylate emulsions.

*Example 10 - Mousse Formulation*

An aqueous mousse composition was prepared from the ingredients shown in Table 14 using a conventional mixing technique.

*Table 14- Mousse Formulation*

| Ingredient | Control Weight % | Silicone Acrylate Emulsion Weight % | Silicone Acrylate Emulsion Weight % | Silicone Acrylate Emulsion Weight % | PVP/VA Weight % |
|---|---|---|---|---|---|
| Deionized Water | q.s. to 100 | q.s. to 100 | q.s. to 100 | q.s. to 100 | q.s. to 100 |
| Cocoamidopropylbetaine[1] | 0.5 | 0.5 | 0.5 | 0.5 | 0.5 |
| PEG-150 Distearate [2] | 0.3 | 0.3 | 0.3 | 0.3 | 0.3 |
| C[3] | | 13.3 | | | |
| D[4] | | | 13.2 | | |
| PVP/VA Copolymer[5] | | | | | 4.0 |
| DMDM Hydantoin[6] | 0.2 | 0.2 | 0.2 | 0.2 | 0.2 |
| 1. Monoteric CAB-LC® available from Uniqema of New Castle, DE<br>2. Lipopeg 6000-DS available from Lipo Chemicals, Inc. of Paterson, NJ<br>3. C. concentration based on 2% active silicone acrylate level. (30.1% Active)<br>4. D. concentration based on 2% active silicone arrylate level, (30.3% Active)<br>5. PVP/VA E-735 available from International Specialty Products of Wayne. NJ<br>6. Glydant® available from Lonza. Inc. of Fairlawn. NJ | | | | | |

**Table 15- Curl Retention Results for Hair Mousse Formulations**

| Formulation | Curl Retention (%) after 5 hrs | Observations |
|---|---|---|
| C | 55 | Well defined curl, soft feel, easy to comb, some spring |
| D | 55 | Well defined curl, soft feel, easy to comb some spring |
| PVP/VA | 61 | Well defined curl, hard to comb, some spring |

The curl retention test results shown in Table 15 indicate that the mousse formulations containing the silicone-acrylate emulsions of the present invention are useful for conditioning hair and for providing additional flexible styling and hold benefits. The tresses were also much easier to comb compared to the tresses treated with the mousse containing the PVPNA copolymer.

*Example 11 - Hair Gel Formulation*

A styling gel composition was prepared from the ingredients shown in Table 16.

*Table 16- Hair Gel Formulation*

| Ingredient | Control Weight % | Silicone Acrylate Emulsion Weight % | Silicone Acrylate Emulsion Weight % | Silicone Acrylate Emulsion Weight % | PVP/VA Weight % |
|---|---|---|---|---|---|
| Deionized Water | q.s. to 100 | q.s. to 100 | q.s. to 100 | q.s. to 100 | q.s. to 100 |
| Acrylates/C10-30 alkyl Acrylate Crosspolymer (2% soln)[1] | 25 | 25 | 25 | 25 | 25 |
| Triethanolamine[2] | | | | | |
| PEG-60 Hydrogenated Castor Oil[3] | 1.0 | 1.0 | 1.0 | 1.0 | 1.0 |

(continued)

| Ingredient | Control Weight % | Silicone Acrylate Emulsion Weight % | Silicone Acrylate Emulsion Weight % | Silicone Acrylate Emulsion Weight % | PVP/VA Weight % |
|---|---|---|---|---|---|
| C[4] | | 13.3 | | | |
| D[5] | | | 13.2 | | |
| PVP/VA Copolymer[6] | | | | | 4.0 |
| DMDM Hydantoin[7] | 0.4 | 0.4 | 0.4 | 0.4 | 0.4 |

1. Carbopol ETD2020 available from Noveon Inc. of Cleveland. OH
2. Triethanolamine Care available from BASF of Mount Olive. NJ
3. Cremophor CO 60 available from BASF of Mount Olive. NJ
4. C. concentration based on 2% active silicone acrylate level. (30.1% Active)
5. D. concentration based on 2% active silicone acrylate level. (30.3% Active)
6. PVP/VA E-735 available from International Specialty Products of Wayne. NJ
7. Glydant® available from Lonza. Inc. of Fairlawn. NJ

**Table 17- Curl Retention Results for Hair Gel Formulations**

| Formulation | Curl Retention (%) after 5 hrs | Observations |
|---|---|---|
| Control | 29.6 | Difficult to comb, some spring back, loose curl |
| C | 33.9 | Easy to comb, good spring back, well defined curl |
| D | 31.0 | Easy to comb, good spring back, well defined curl |
| PVP/VA | 36.4 | Very difficult to comb, some spring back, well defined curl |

The curl retention test results for the styling gels containing the silicone-acrylate emulsions shown in Table 17 demonstrate similar curl retention properties to the organic styling resin PVP/VA; however the tresses are easier to comb and have more spring back properties when the tress is extended to full length.

*Example 12 - Shower Gel Application*

This example illustrates G evaluated in shower gel formulations and compared with the control which does not contain any silicone emulsion.

*Test procedure*

The Shower gel sensory evaluation is designed to compare several benefits such as foaming, wet skin feel and after feel between two shower gels, by paired comparison, using a human panel.

The panelist rinses the left and right hand and their respective forearms for 8 seconds. Three ml of each shower gel are applied on each hand and the panelist lathers both forearms for 20 seconds. After this lathering period, the panelist compares the foam generation, quality and quantity. After that the panelist rinses his/her right and left arms for 20 seconds and then compares the ease of rinsing and the slipperiness. The panelist dries both forearms gently and evaluates the tackiness just after padding and finally the smoothness, film presence and suppleness after an additional two minute wait period.

*Table 18 -SHOWER GEL*

| Ingredients | Control | Emulsion G |
|---|---|---|
| | 13 | 31 |
| Phase A | | |
| Empicol ES B-3 | 30% | 30% |

(continued)

| | | |
|---|---|---|
| Phase A | | |
| Oramix NS 10 | 5% | 5% |
| Amonyl 380 BA | 10% | 10% |
| Phase B | | |
| Brij 30 | 2% | 2% |
| Sepigel 305 | 2% | 2% |
| G | | 5% active (12.9%) |
| Phase C | | |
| Water | 51% | 38.1% |
| Citric acid (50%) to pH 6 | qs | qs |
| Viscosity after 24 h | 630 cps | 260cps |

*Formulation process*

The shower gel is prepared by first mixing Empicol ESB-3, Oramix NS 10, and Amonyl 380 BA together (phase A) and then mixing Brij 30 and Sepigel 305 together (phase B). Phase A is added to phase B. The water from phase C is added slowly and then the silicone-acrylate emulsion prototype (G) is added. The pH is adjusted to 6.

In the above Table 18 the Empicol ESB-3, Oramix NS 10, Amonyl 380 BA and Sepigel 305 are sold by Seppic and Brij 30 is sold by Uniqema. The Empicol ESB-3 is sold by Albright & Wills.

*SHOWER GEL*

Sensory testing for the shower gel evaluation was performed on formulations paired as control vs. Emulsion G according to ASTM Standards E 1958-98 (Standard Guide for Sensory Claim Substantiation) and E 253 (Terminology Relating to Sensory Evaluation of Materials and Products), and ISO Standard 6658 (Sensory Analysis - Methodology - General Guidance). The results are reported in Table 19. The test was performed on 12 panelists. The difference is recorded in the last row with the corresponding confidence level.

*Table 19- results of shower gel sensory evaluation*

| | foam generation | foam quality | foam quantity | ease of rinse | slipperiness | tackiness | smoothness | film presence | suppleness |
|---|---|---|---|---|---|---|---|---|---|
| control | 6 | 6 | 2 | 4 | 4 | 6 | 7 | 5 | 4 |
| G | 6 | 6 | 10 | 8 | 8 | 6 | 5 | 7 | 8 |
| difference | no | no | 95% | no | no | no | no | no | no |

The shower gel containing the prototype silicone-organic emulsion, G, gave an increase in foam quantity (95 % confidence level) vs. the control.

*Example 13 - Lipstick Application*

The evaluation of the lipstick is designed to provide the benefits brought by the lipstick such as ease of application, shine just after application and after 1 hour and its non transfer. This comparison is done by pair comparison with a human panel. A minimum of 18 panelists is required for one comparison of 2 formulations.

*Test procedure*

1. Five minutes before testing, the panelists must wash their hands and forearms with a silicone free soap and dry them with a paper towel. To determine *the ease of application* the panelist is asked to apply the lipsticks by making one line of each on the back of their non-dominant hand and to indicate the <u>easiest product to apply</u>.
2. *The shine and color are assessed right after application using the following method:* the operator applies the lipstick on the volar forearm of the panelist by making 3 lines of each (pre-cut paper with 2 holes of 5cm length and 8mm width - in accordance with the size of the stick). The panelist indicates the <u>shiniest product</u> and the <u>darkest product</u> between the two lipsticks assessed.
3. T*he shine and the color are assessed 1 hour after application* by the panelist indicating the <u>shiniest product</u> and the <u>darkest product</u> between the two lipsticks assessed.
4. To assess *the non-transfer* the operator applies a glass microscope slide on the 2 spots of lipstick for 10 sec. using equivalent pressure on both spots. The panelist then indicates which product has <u>transferred</u> less lipstick onto the microscope slide.

*Table 20 and 20' - Lipstick composition by weight*

| | **control 25% water** | | I | | H | |
|---|---|---|---|---|---|---|
| **Ingredients** | weight % | | weight% | g | weight% | g |
| ***Phase A*** | | | | | | |
| Lauryl PEG/PPG-18/18 Methicone[1] | 3% | 0.9 | 3% | 0.9 | 3% | 0.9 |
| Cyclopentasiloxane[2] | 17% | 5.1 | 17% | 5.1 | 17% | 5.1 |
| Ozokerite wax | 9.5% | 2.85 | 9.5% | 2.85 | 9.5% | 2.85 |
| Candelilla wax | 17.10% | 5.13 | 17.10% | 5.13 | 17.10% | 5.13 |
| Vaselinum | 1.50% | 0.45 | 1.50% | 0.45 | 1.50% | 0.45 |
| Oleyl alcohol | 3.40% | 1.02 | 3.40% | 1.02 | 3.40% | 1.02 |
| Avocado oil | 1.40% | 0.42 | 1.40% | 0.42 | 1.40% | 0.42 |
| Octyl dodecanol | 2.10% | 0.63 | 2.10% | 0.63 | 2.10% | 0.63 |
| Castor Oil | 10.0% | 3 | 10.0% | 3 | 10.0% | 3 |
| Pigment blend** | 10% | 3 | 10% | 3 | 10% | 3 |
| ***Phase B*** | | | | | | |
| Water | 25.0% | 7.5 | | | 1.30% | 0.39 |
| H | | | 25% | 7.5 | | |
| G | | | | | 23.70% | 7.1 |
| | 100% | 30 | 100% | 30 | 100% | 30 |
| 1. Dow Corning® 5200 Formulation aid available from Dow Corning, Midland, MI<br>2. Dow Corning® 245 Fluid available from Dow Corning, Midland, MI | | | | | | |

In the above table the ozokerite wax is sold by Baerlocher France, the candelilla wax is sold by A & E Connock, the vaselinum is sold by Aiglon, the oleyl alcohol is sold by Croda, the avocado oil is sold by Alban Muller International, the octyl dodecanol is sold by Henkel-Cognis, and the castor oil is sold by Aldrich.

*Table 20'*

| **Pigment blend**** | weight% | g |
|---|---|---|
| Cyclopentasiloxane[1] | 77.50% | 7.75 |
| Covasil Ti O2 | 5% | 0.5 |
| Covasil Red W 3801 | 17.50% | 1.75 |
| | 100% | 10 |
| 1. Dow Corning® 245 Fluid available from Dow Corning, Midland, MI | | |

In the above table the Covasil TiO2 and Covasil Red W 3801 were supplied by LCW.

*Formulation process:*

The pigment blend is first prepared by blending the Covasil Titanium dioxide with the Covasil red W 3801 in cyclopentasiloxane and mixed with high shear until the mixture is homogeneous. The ingredients from phase A are loaded into a separate beaker. The ingredients from phase B are loaded into a separate beaker, and the water phase and the silicone acrylate emulsion are then added slowly. Phase A is heated with a water bath at 80°C and is mixed at 400 RPM until homogeneous. Phase B is heated at 60°C and is added dropwise to phase A at 600 RPM with constant mixing for 3 minutes after all phase B is added. Pour the final lipstick formulation into a metal stick and keep it refrigerated for one hour to solidify it.

*Table 21 -Paired comparison for lipstick application*

| **parameter** | **number panelists** | **score control** | **score H** | **difference** |
|---|---|---|---|---|
| ease of application | 14 | 10 | 4 | no |
| shine just after application | 14 | 2 | 12 | 95% |
| color just after application | 14 | 12 | 2 | 95% |
| shine 1h after application | 14 | 2 | 12 | 95% |
| color 1h after application | 14 | 9 | 5 | no |
| no transfer | 14 | 9 | 5 | no |

Sensory testing for shine attributes is performed on formulations paired as control vs. H according to ASTM Standards E 1958-98 (Standard Guide for Sensory Claim Substantiation) and E 253 (Terminology Relating to Sensory Evaluation of Materials and Products), and ISO Standard 6658 (Sensory Analysis- Methodology- General Guidance). The results are reported in Table 21. The difference is recorded in the last column with the corresponding confidence level.

This example illustrates that emulsion H exhibits superior shine than the control formulation just after application and one hour after application.

*Example 14 - Wash-Off Resistance*

The substantivity or wash-off resistance of silicone on skin is evaluated versus time or washes in order to measure the long lasting effect of a formulation or a product. The silicone presence is detected and quantified by infrared spectroscopy. It is assessed on a human panel: a minimum of 3 panelists is required for one evaluation.

*Test method procedure:*

Before testing, the panelists must wash their hands and forearm with a silicone free soap and dry them with a paper towel. Then a first spectrum of neat skin is taken in order to have a skin control area (blank) and to see if there is

any contamination of the skin. A rectangle of 9 x 4 cm is drawn on the volar forearm of the panelist. If the amide and water peaks do not appear clearly, wet the forearm with a humid tissue and then wipe it with a dry tissue until no excess water remains. This will increase the contact of the skin with the crystal. Next, 0.1g or 100μl of the product is applied onto the outlined site and rubbed in with a spatula until absorption, making sure to use the whole surface of the application site. After 15 minutes a second Infrared spectrum is taken. The operator washes the test site with 3ml of a 0.5% sodium lauryl ether sulfate (SLES) solution: wet the skin 5 sec under 37°C tap water, apply the SLES, and rub it 15 times over the test site. The panelist rinses the test site by quickly passing the arm 10 times under 37°C tap water and carefully patting the test site with a dry tissue until no water remains. A third spectrum is taken after this first wash (wash 1). Repeat the washing step a second time and take a spectrum (wash 2). Repeat the washing step until three different washes and three spectra are completed. The ratio of peaks measured is equal to the height of the silicone peak divided by the height of the amide peak. The skin spectra of the untreated skin are subtracted and the corresponding percentage of product left over is calculated. The silicone left on the skin is quantified by FTIR at time 0 (before any wash), after 1 wash, 2 washes and 3 washes. The wash off resistance was improved for the silicone-acrylate emulsion sample I.

In a first aspect, a personal care composition comprising a silicone polymer and organic polymer containing alloy and /or hybrid emulsion is disclosed.

In a second aspect, the personal care composition of the first aspect is disclosed, wherein the emulsion is obtained by (i) preparing an emulsion containing a linear silicone polymer by emulsion polymerization in which (a) the ring of a cyclic siloxane oligomer is opened, (b) an hydroxy endblocked siloxane oligomer is condensed, using an acid or base catalyst in the presence of water, or in which (c) an hydrogen endblocked siloxane oligomer and a vinyl endblocked siloxane oligomer are reacted by hydrosilylation using a catalyst; (ii) adding to the emulsion in (i) components for preparing an emulsion containing an organic polymer by free radical emulsion polymerization of one or more ethylenically unsaturated organic monomers; and (iii) heating the emulsion from (ii).

In a third aspect, the personal care composition of the second aspect is disclosed, wherein the ethylenically unsaturated organic monomer is an acrylate ester, a methylacrylate ester, a fluorinated acrylate, a fluorinated methacrylate, acrylic acid, methacrylic acid, allyl methacrylate, dimethylaminoethyl methacrylate, a vinyl halide, a vinyl ester, a vinyl aromatic compound, a vinyl ester of a monocarboxylic acid, or a vinyl pyrrolidone.

In a fourth aspect, the personal care composition of the second aspect is disclosed, wherein the components in (ii) comprise one or more organic monomers and a free radical indicator, and the components are added to the emulsion in (i) separately.

In a fifth aspect, the personal care composition of the second aspect is disclosed, wherein the components in (ii) comprise one or more organic monomers and a free radical initiator, and the components are added to the emulsion in (i) simultaneously.

In a sixth aspect, the personal care composition of the second aspect is disclosed, wherein the silicone polymer in (i) comprises a linear siloxane free of trifunctional T units $RSiO_{3/2}$ and tetrafunctional Q units $SiO_{4/2}$ capable of providing crosslinking of the silicone polymer or the reaction of the silicone polymer with the organic polymer; the organic polymer comprises a polymer free of silicon atoms; and the resulting emulsion is an aqueous emulsion containing an immiscible mixture of linear silicone polymers and organic polymers.

In a seventh aspect, the personal care composition of the second aspect is disclosed, wherein the ethylenically unsaturated organic monomer is selected from the group consisting of butyl acrylate, methyl acrylate, methyl methacrylate, methacrylic acid, allyl methacrylate, dimethylaminoethyl methacrylate, 2-ethylhexyl acrylate, vinyl acetate, vinyl esters of monocarboxylic acids, vinyl pyrrolidone, and styrene.

In a eighth aspect, the personal care composition of the second aspect is disclosed, wherein the silicone polymer emulsion in (i) contains silicone polymer particles having an average particle diameter of 30-500 nanometer, and the viscosity of the phase containing the silicone polymer is from 2,000 to 10,000,000 $mm^2/s$ at 25°C.

In a ninth aspect, the personal composition of the first aspect is disclosed, wherein the silicone polymer and organic polymer containing alloy and/or hybrid emulsion composition is obtained by (i) preparing a first emulsion containing a silicone polymer by emulsion polymerization in which (a) the ring of a cyclic siloxane oligomer is opened, in which (b) an hydroxy endblocked siloxane oligomer is condensed, using an acid or base catalyst in the presence of water, or in which (c) an hydrogen endblocked siloxane oligomer and a vinyl endblocked siloxane oligomer are reacted by hydrosilylation using a catalyst; (ii) preparing a second emulsion containing an organic polymer by free radical emulsion polymerization of an ethylenically unsaturated organic monomer; and combing the first and second emulsions.

In a tenth aspect, the personal care composition of the ninth aspect is disclosed, wherein the ethylenically unsaturated organic monomer is an acrylate ester, a methylacrylate ester, a vinyl halide, a vinyl ester, or a vinyl aromatic compound.

In an eleventh aspect, the personal care composition of the ninth aspect is disclosed, wherein the silicone polymer in (i) comprises a linear siloxane free of trifunctional T units $RSiO_{3/2}$ and tetrafunctional Q units $SiO_{4/2}$ capable of providing crosslinking of the silicone polymer or the reaction of the silicone polymer with the organic polymer; the organic polymer comprises a polymer free of silicon atoms; and the resulting emulsion is an aqueous emulsion containing

an immiscible mixture of linear silicone polymers and organic polymers.

In a twelfth aspect, the personal care composition of the ninth aspect is disclosed, wherein the ethylenically unsaturated organic monomer is selected from the group consisting of butyl acrylate, methyl acrylate, methyl methacrylate, methacrylic acid, allyl methacrylate, dimethylaminoethyl methacryate, 2-ethylhexyl acrylate, vinyl acetate, vinyl esters of monocarboxylic acids, vinyl pyrrolidone, and styrene.

In a thirteenth aspect, the person care composition of the ninth aspect is disclosed, wherein the first emulsion contains silicone polymer particles having an average particle diameter of 30-500 nanometers, and the viscosity of the phase containing the silicone polymer is from 2,000 to 10,000,000 $mm^2/s$ at 25 °C.

In a fourteenth aspect, the composition of the first aspect is disclosed, wherein the person care composition is selected from a colour cosmetic, a lipstick, a foundation, an eye shadow, a mascara, a shampoo, a hair conditioner, a hair fixative, a hair styling aid, a hair colorant, a hair relaxer, a shower gel, a skin moisturizer, a body conditioner, a sun protection product, and antiperspirant, and a deodorant.

## Claims

**1.** A process for preparing a silicone-organic alloy or hybrid emulsion comprising:

(i) preparing an emulsion containing an aminofunctional silicone polymer by emulsion polymerization in which (a) the ring of a cyclic siloxane oligomer is opened using an acid or base catalyst in the presence of water and an aminofunctional silane, or (b) a hydroxy endblocked siloxane oligomer is condensed, using an acid or base catalyst in the presence of water and an aminofunctional silane,
(ii) adding to the emulsion in (i) components for preparing an emulsion containing an organic polymer by free radical emulsion polymerization of one or more ethylenically unsaturated organic monomers; and
(iii) heating the emulsion from (ii).

**2.** A process for preparing a silicone-organic alloy or hybrid emulsion comprising:

(i) preparing a first emulsion containing an aminofunctional silicone polymer by emulsion polymerization in which (a) the ring of a cyclic siloxane oligomer is opened using an acid or base catalyst in the presence of water and an aminofunctional silane, or (b) an hydroxy endblocked siloxane oligomer is condensed, using an acid or base catalyst in the presence of water and an aminofunctional silane;
(ii) preparing a second emulsion containing an organic polymer by free radical emulsion polymerization of an ethylenically unsaturated organic monomer; and combining the first and second emulsions.

**3.** The process of claim 1 or 2 where the aminofunctional silane is (ethylenediaminepropyl) trimethoxy silane.

**4.** The emulsion prepared according to claims 1, 2, or 3.

**5.** A personal care composition comprising the emulsion of claim 4.

## Patentansprüche

**1.** Ein Verfahren zum Herstellen eines silikonorganischen Gemischs oder einer Hybrid-Emulsion, das Folgendes beinhaltet:

(i) Herstellen einer ein aminofunktionelles Silikonpolymer enthaltenden Emulsion durch Emulsionspolymerisation, wobei (a) der Ring eines zyklischen Siloxanoligomers unter Verwendung eines Säure- oder Basekatalysators in Gegenwart von Wasser und einem aminofunktionellen Silan geöffnet wird oder (b) ein hydroxyendgeblocktes Siloxanoligomer unter Verwendung eines Säure- oder Basekatalysators in Gegenwart von Wasser und einem aminofunktionellen Silan kondensiert wird,
(ii) Hinzufügen zu der Emulsion in (i) von Komponenten zur Herstellung einer Emulsion, die ein organisches Polymer enthält, durch Emulsionspolymerisation freier Radikale von einem oder mehreren ethylenisch ungesättigten organischen Monomeren; und
(iii) Erhitzen der Emulsion aus (ii).

**2.** Ein Verfahren zum Herstellen eines silikonorganischen Gemischs oder einer Hybrid-Emulsion, das Folgendes beinhaltet:

(i) Herstellen einer ein aminofunktionelles Silikonpolymer enthaltenden ersten Emulsion durch Emulsionspolymerisation, wobei (a) der Ring eines zyklischen Siloxanoligomers unter Verwendung eines Säure- oder Basekatalysators in Gegenwart von Wasser und einem aminofunktionellen Silan geöffnet wird oder (b) ein hydroxyendgeblocktes Siloxanoligomer unter Verwendung eines Säure- oder Basekatalysators in Gegenwart von Wasser und einem aminofunktionellen Silan kondensiert wird;
(ii) Herstellen einer zweiten ein organisches Polymer enthaltenden Emulsion durch Emulsionspolymerisation freier Radikale eines ethylenisch ungesättigten organischen Monomers; und In-Verbindung-Bringen der ersten und zweiten Emulsion.

**3.** Verfahren gemäß Anspruch 1 oder 2, wobei das aminofunktionelle Silan (Ethylendiaminpropyl)trimethoxysilan ist.

**4.** Die Emulsion, hergestellt gemäß den Ansprüchen 1, 2 oder 3.

**5.** Eine Körperpflegezusammensetzung, die die Emulsion gemäß Anspruch 4 beinhaltet.

## Revendications

**1.** Un procédé pour préparer un alliage silicone-organique ou une émulsion hybride comprenant :

(i) préparer une émulsion contenant un polymère de silicone aminofonctionnel par polymérisation en émulsion dans laquelle (a) le cycle d'un oligomère siloxane cyclique est ouvert en utilisant un catalyseur acide ou basique en présence d'eau et d'un silane aminofonctionnel, ou (b) un oligomère siloxane à blocage terminal hydroxy est condensé, en utilisant un catalyseur acide ou basique en présence d'eau et d'un silane aminofonctionnel,
(ii) ajouter à l'émulsion en (i) des composants pour préparer une émulsion contenant un polymère organique par polymérisation en émulsion en présence de radicaux libres d'un ou de plusieurs monomères organiques éthyléniquement insaturés ; et
(iii) chauffer l'émulsion de (ii).

**2.** Un procédé pour préparer un alliage silicone-organique ou une émulsion hybride comprenant :

(i) préparer une première émulsion contenant un polymère de silicone aminofonctionnel par polymérisation en émulsion dans laquelle (a) le cycle d'un oligomère siloxane cyclique est ouvert en utilisant un catalyseur acide ou basique en présence d'eau et d'un silane aminofonctionnel, ou (b) un oligomère siloxane à blocage terminal hydroxy est condensé, en utilisant un catalyseur acide ou basique en présence d'eau et d'un silane aminofonctionnel ;
(ii) préparer une deuxième émulsion contenant un polymère organique par polymérisation en émulsion en présence de radicaux libres d'un monomère organique éthyléniquement insaturé ; et combiner les première et deuxième émulsions.

**3.** Le procédé de la revendication 1 ou de la revendication 2 où le silane aminofonctionnel est un silane triméthoxy (éthylènediaminepropyl).

**4.** L'émulsion préparée selon les revendications 1, 2, ou 3.

**5.** Une composition de soins d'hygiène personnelle comprenant l'émulsion de la revendication 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005056682 A
- US 2891920 A
- US 3294725 A
- US 4999398 A
- US 5502105 A
- US 5661215 A
- US 6316541 B
- US 6051216 A
- US 5919441 A
- US 5981680 A
- WO 2004060271 A
- WO 2004060101 A
- WO 2004060276 A
- WO 03105801 A
- US 20030235553 A
- US 20030072730 A
- US 20030170188 A
- EP 1266647 A
- EP 1266648 A
- EP 1266653 A
- WO 03105789 A
- WO 2004000247 A
- WO 03106614 A
- WO 2004054523 A
- US 20040180032 A
- WO 2004054524 A
- US 4009256 A
- GB 9403156 A
- WO 9522311 A
- US 3962418 A
- US 3958581 A
- US 4741855 A
- US 4788006 A
- US 4704272 A
- US 2798053 A
- EP 678292 A
- US 4585597 A
- FR 2236515
- FR 2282426
- FR 2645148
- FR 2430938
- FR 2592380
- FR 2326405 A
- FR 2440933 A
- EP 0114607 A
- EP 0487404 A
- EP 0518772 A
- EP 0518773 A
- US 6013682 A
- WO 03101412 A2
- US 4122029 A, Gee
- US 5387417 A, Rentsch
- US 5811487 A, Schulz
- US 5389364 A
- US 5409695 A
- US 5419627 A
- US 5504149 A


### Non-patent literature cited in the description


- Chemical Engineer's Handbook. 1973
- CTFA Cosmetic Ingredient Directory
- CTFA. Cosmetic, Toiletry, and Fragrance Association